(19) Europäisches Patentamt
European Patent Office
Office européen des brevets

(11) **EP 2 155 854 B1**

(12) **EUROPÄISCHE PATENTSCHRIFT**

(45) Veröffentlichungstag und Bekanntmachung des Hinweises auf die Patenterteilung:
**11.01.2017 Patentblatt 2017/02**

(51) Int Cl.:
*C12M 1/107* *(2006.01)*   *C12M 1/12* *(2006.01)*
*B01J 8/02* *(2006.01)*

(21) Anmeldenummer: **08759655.7**

(22) Anmeldetag: **16.05.2008**

(86) Internationale Anmeldenummer:
**PCT/EP2008/056005**

(87) Internationale Veröffentlichungsnummer:
**WO 2008/142007 (27.11.2008 Gazette 2008/48)**

(54) **FERMENTER ZUR ERZEUGUNG VON BIOGAS AUS PUMPBAREM ORGANISCHEN MATERIAL**

FERMENTER FOR GENERATING BIOGAS FROM PUMPABLE ORGANIC MATERIAL

FERMENTEUR DESTINÉ À LA PRODUCTION DE BIOGAZ À PARTIR D'UN MATÉRIAU ORGANIQUE SUSCEPTIBLE D'ÊTRE POMPÉ

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **23.05.2007 DE 102007024378**

(43) Veröffentlichungstag der Anmeldung:
**24.02.2010 Patentblatt 2010/08**

(73) Patentinhaber:
• **Gantefort, Wilhelm**
**46359 Heiden (DE)**
• **Beck, Jürgen**
**72108 Rottenburg (DE)**

(72) Erfinder:
• **GANTEFORT, Wilhelm**
**46359 Heiden (DE)**

• **BECK, Jürgen**
**72108 Rottenburg (DE)**

(74) Vertreter: **Michalski Hüttermann & Partner Patentanwälte mbB**
**Speditionstraße 21**
**40221 Düsseldorf (DE)**

(56) Entgegenhaltungen:
**EP-A- 0 335 825       WO-A-2007/054193**
**DE-A1- 3 604 415      DE-A1- 3 608 466**
**DE-A1- 3 715 952      DE-A1- 4 415 017**
**DE-A1- 19 756 485     DE-A1-102005 012 936**
**DE-C1- 19 805 580     DE-U1- 29 521 085**
**JP-A- 2005 081 238    US-A- 4 532 042**

EP 2 155 854 B1

**Beschreibung**

Die vorliegende Erfindung betrifft ein <u>Verfahren</u> zur Erzeugung von Biogas aus pumpbarem organischen Material mit geringem Anteil an organischer Trockensubstanz (oTS) gemäss dem vorliegenden Anspruch 1, <u>sowie einen Fermenter geeignet zur Durchführung des Verfahrens Anspruch 1.</u>

Fermenter zur Erzeugung von Biogas stoß0en auf zunehmendes Interesse, seit die Diskussion um erneuerbare Energien sowie deren Förderung mehr und mehr in den Mittelpunkt der öffentlichen Aufmerksamkeit gerückt ist. Solche Fermenter sind aus dem Einsatz in landwirtschaftlichen Betrieben sowie kommunalen Kläranlagen bekannt. Prinzip dieser Fermenter ist, dass organisches Material in einem geschlossenen Behälter gelagert wird und die enthaltenen organischen Kohlenstoffverbindungen durch mikrobielle Aktivität zu Methangas abgebaut werden, welches aufgefangen und zur Wärme- und/oder Stromerzeugung verwendet wird. Die auf diese Weise gewonnene Energie ist nahezu $CO_2$-neutral, da das bei der Verbrennung freigesetzte Kohlendioxid zuvor durch pflanzliche Fotosynthese der Atmosphäre entnommen wurde.

Im Folgenden soll zunächst der unter Sauerstoffabschluss verlaufende Gärprozess zur Erzeugung von Biogas beschrieben werden. Der gesamte Gärprozess kann in mehrere Phasen eingeteilt werden. In der ersten Phase werden die im zu vergärenden Substrat enthaltenen Kohlenhydrate, Fette und Proteine durch fakultativ und obligat anaerobe Mikroorganismen in niedermolekulare Kohlenwasserstoffverbindungen ($C_1$-$C_5$-Körper) aufgespalten. Kohlenhydrate werden dabei sukzessive zu Propionsäure oder Buttersäure bzw. Butanol abgebaut, Fettsäuren über den Weg der $\beta$-Oxidation schrittweise in $C_2$-Einheiten zerlegt, die als Essigsäure freigesetzt werden, und Aminosäuren nach der Stickland - Reaktion gekoppelt zu Essigsäure, Ammoniak und $CO_2$ abgebaut.

Diese Zwischenprodukte werden wiederum zu den methanogenen Substraten Essigsäure ($CH_3COOH$), Wasserstoff ($H_2$), Kohlensäure ($H_2CO_3$), Ameisensäure ($HCOOH$) und Methanol ($CH_3OH$) abgebaut. Diese methanogenen Substrate werden wiederum von obligat anaeroben, methanbildenen (methanogenen) Bakterien der Gattungen *Methanobacterium, Methanosarcina* und *Methanospirillum* in den folgenden Reaktion zu Methan, Kohlendioxid und Wasser abgebaut:

$$1) \qquad CH_3COO^- + H^+ \text{------>} CH_4 + CO_2$$

$$2) \qquad HCO_3^- + H^+ + 4H_2 \text{- - >} CH_4 + 3\ H_2O$$

$$3) \qquad HCOO^- + H^+ + 3H_2 \text{- - >} CH_4 + 2\ H_2O$$

$$4) \qquad CH_3OH + H_2 \text{------ >} CH_4 + H_2O$$

Das in Reaktion 2) erwähnte $HCO_3^-$ entsteht dabei durch Lösung von Kohlendioxid im Wasser nach folgender Gleichung:

$$5) \qquad H_2O + CO_2 \text{------>} HCO_3^- + H^+.$$

Über 70% des Methans entstehen über die Spaltung von Essigsäure, also über Reaktion 1. Da es sich bei der Biogasvergärung um einen Mischprozess handelt, bei dem in den verschiedenen Phasen verschiedene Mikroorganismen aktiv sind, muss den unterschiedlichen Ansprüchen aller Mikroorganismen Rechnung getragen werden, um einen möglichst hohen Ertrag zu erzielen. Ausschlaggebend sind jedoch die für die Aktivität der methanogenen Bakterien erforderlichen Bedingungen. Letztere benötigen aufgrund ihrer Eigenschaften als obligate Anaerobier ein strikt sauerstoffreies Milieu. Überdies bevorzugen sie einen leicht alkalischen pH-Wert.

Aus der DE 197 564 85 ist ein Faulbehälter mit Rührwerk zum Einsatz in landwirtschaftlichen Biogasanlagen und kommunalen Kläranlagen bekannt. Dieser weist eine runde Bodenfläche, einen Füllstutzen und ein am Umfang des Faulbehälters angebrachtes Rührwerk mit einer Antriebsachse auf. Das Rührwerk ist in einem unterhalb des Füllstutzens angeordneten Rührrohr untergebracht. Das Rührrohr verläuft bevorzugt senkrecht. Der Inhalt des Gärbehälters wird über eine Wandheizung temperiert. Zu vergärendes Substrat wird Über einen relativ weit oben angeordneten Füllstutzen in den Gärbehälter eingebracht, während über einen sehr viel weiter unten angeordneten Ablauf ausgegorenes, weiter unten im Behälter befindliches Material abgepumpt und in ein Gärrückstandslager verbracht wird.

Die in einem solchen Fermenter verwendbaren Substrate müssen einen relativ hohen Anteil an organischer Trockensubstanz (oTS) haben. So weisen z.B. Energiepflanzen wie Mais oder Weizen einen oTS-Anteil von mehr als 60 Gew.-% auf. Mit solchen Energiepflanzen lassen sich bei verhältnismässig kleinen Fermentervolumina hohe Biogasausbeuten erzielen, da sich mit diesen Pflanzen hohe Raumbelastungen einstellen lassen.

Die Größe "Raumbelastung" ist ein Maß für die biologische Belastung eines Fermenters. Für eine konventionelle Biogasanlage ist eine Raumbelastung von 2 bis 5 kg $oTS/m^3$ pro Tag anzustreben. Unter einer Raumbelastung von 2

kg oTs/m$^3$ pro Tag spricht man von Schwachlast. Eine Raumbelastung von mehr als 5 kg oTs/m$^3$ pro Tag wird als Hochlast bezeichnet.

DE3604415 offenbart ein mehrstufiges Verfahren zur Umwandlung von organischen und anorganischen Stoffen durch Katalysatoren, dadurch gekennzeichnet, dass der Reaktor zylinderförmig ausgelegt ist, weitere Zylinder konzentrisch im Reaktor angeordnet sind und separate Stufen bilden, die Stufen vertikal durchströmt werden, die Stufen nacheinander und abwechselnd von oben nach unten und von unten nach oben durchströmt werden, die von unten nach oben durchströmten Stufen am oberen Ende einen Überlauf zur nächsten Stufe besitzen, die von oben nach unten durchströmten Stufen am unteren Ende Überströmkanäle zur nächsten Stufe aufweisen, nach der letzten Stufe ggf. in einem kombinierten Apparat eine Entgasung und eine Abtrennung der festen Stoffe stattfindet, wobei diese in die letzte Stufe zurückgeführt werden können, die einzelnen Stufen sowie der kombinierte Entgaser - Abtrenner auf Grund von geringer werdenden Flüssigkeitshöhen selbsttätig durchströmt werden, sowie heterogen zunehmende Stoffumwandlung beim Durchlaufen des Reaktors und heterogene Entgasung beim Durchlaufen des Entgaserraumes stattfindet.

EP0335825A1 offenbart ein Verfahren zur zweistufigen anaeroben Aufbereitung flüssiger Substrate mit hoher organischer Belastung in einem einzigen Reaktor mit zwei miteinander verbundenen Kammern, in deren erster die Hydrolyse und die saure Gärung (Gärkammer) und in deren zweiter die Methanbildung (Faulkammer) stattfindet, wobei die Einleitung des Substrates in die Faulkammer im unteren Bereich der Faulkammer erfolgt, wobei ferner sedimentierte Feststoffe im Bodenbereich des Reaktors abziehbar sind, wobei außerdem der Abwasseranteil des behandelten Substrates oben aus der Faulkammer abgeleitet wird und wobei das gebildete Gärgas und das Faulgas oberhalb des Flüssigkeitsspiegels abgezogen werden, wobei das Substrat der Gärkammer im Abstand vom Boden der Gärkammer in einer Höhe von maximal einem Drittel des Flüssigkeitsspiegels der Gärkammer zugeführt wird, die im Substrat enthaltenen Feststoffe und gebildeten Zersetzungsprodukte in der Gärkammer weitgehend sedimentiert und als Gärschlamm gesondert abgezogen werden und nur das von Feststoffen weitgehend befreite Substrat aus dem oberen Teil der Gärkammer in die Faulkammer eingeleitet wird, wobei die Einleitung im Abstand vom Boden der Faulkammer in einer Höhe von maximal einem Drittel des Flüssigkeitsspiegels der Faulkammer erfolgt.

DE4415017 offenbart einen Zweistufigen Kombi-Biogasreaktor zur Aufbereitung von insbesondere Gülle, der aus einem Biogasreaktor mit einer ersten, im unterenTeil trichterförmig ausgebildeten, Verarbeitungsstufe und einer ringförmigen zweiten Verarbeitungsstufe besteht, die über Überleitungsöffiiungen, in denen sich Gasleiteinrichtungen befinden, miteinander verbunden sind, wobei der obere Teil des Biogasreaktors für eine Gastrennung zu einem CH-Gasraum und einem CO-Gasraum ausgebildet ist, wobei innerhalb der ersten Verarbeitungsstufe zur Einstellung der-Prozeßtemperatur Heizungen und zur Verhinderung der Schwimmdeckenbildung eine Durchmischung angeordnet sind, wobei in der zweiten Verarbeitungsstufe zwischen der ersten Verarbeitungsstufe und der Reaktorinnenwand ein Anaerobfilter, bestehend aus auf Trägerrahmen montierten Glasfasern, sternförmig angeordnet ist und wobei der untere Teil de rzweiten Verarbeitungsstufe als Schlammbettreaktor ausgebildet ist.

Allerdings eignen sich herkömmliche Biogasfermenter lediglich für Substrate mit hohen oTS-Anteilen, also insbesondere Substrate aus nachwachsenden Rohstoffen (NaWaRo), insbesondere aus sogenannten Energiepflanzen wie z.B. Getreide, Silomais oder Futterrüben.

Substrate mit geringen oTS-Anteilen, wie z.B. Gülle, Gärreste, Schlempe (Rückstände aus der Alkoholvergärung, insbesondere der Bioethanolproduktion), Klärschlämme oder hochbelastete Abwässer aus der Nahrungsmittelindustrie eignen sich nicht für die ausschließliche Verwendung in diesen Fermentern, sondern kommen allenfalls als Impfsubstrat oder in einem Gemisch mit Energiepflanzen-Substraten(d.h. zur Kofermentation) in Frage, da die Menge andaraus pro m$^3$ Faulraumvolumen erzeugbarem Biogas so gering ist, dass damit kaum die für den Betrieb des Fermenters erforderliche Energie (Heizenergie,Strom für den Antrieb der Rührwerke) zurückgewonnen werden kann.

Dies liegt eben daran, dass aufgrund des geringen oTS-Anteils mit diesen Substraten keine hohen Raumbelastungen verwirklicht werden können, ohne massive Auschwemmung von Methanbildnern zu riskieren.

Hinzu kommt, dass herkömmliche Fermenter permanent mit dem Problem der Aufkonzentration von Propionsäure zu kämpfen haben, die ab einer bestimmten Konzentration bakteriostatische und fungistatische Wirkungen hat. Dieses Problem tritt dann auf, wenn bei höherer Raumbelastung die Bildung flüchtiger Fettsäuren wie z. B. Essigsäure schneller vonstatten geht als deren Abbau durch die Methanbildner. Deren Konzentration wird in herkömmlichen Fermentern permanent dazuhin durch Ausschwemmung reduziert und die Reduplikation der methanogenen Bakterien erfolgt mit 10 - 14 Tagen extrem langsam im Vergleich zu den Säurebildnern mit ca. 0,5 - 2 h, so dass es in den herkömmlichen Fermentersystemen sehr schnell zur Versauerung des Fermenterinhalts (gestoppte Methanbildung) und zur Bildung von Propionsäure kommt.

Gülle stellt ein anoxisches System mit relativ hohem pH-Wert dar und ist aus diesem Grunde sehr gut geeignet, in einem Biogasfermenter die für Methanbakterien erforderlichen Bedingungen zu schaffen. Gleichwohl kann die organische Trockensubstanz der Gülle weniger schnell durch Mikroorganismen verstoffwechselt werden als die organische Trockensubstanz aus besagten Energiepflanzen. Dies macht eigentlich eine längere Verweildauer der Gülle in dem Fermenter erforderlich.

So erzeugt ein Rindermastbetrieb mit 400 GV (Großvieheinheiten, 1 GV entspricht 500 kg Lebendgewicht) am Tag etwa 20 m³ Gülle, die behandelt werden müssen. Bei einer Verweildauer von 50 Tagen wäre daher bei herkömmlicher Bauart ein Fermenter mit einem Raumvolumen von 1000 m³ erforderlich.

Rindergülle weist im Mittel einen oTS-Anteil von etwa 6 Gew.-% auf. Bei einer zu erwartenden Ausbeute von 500 m³ Biogas pro Tonne oTS ergibt sich in obigem Beispiel gemäß folgender Gleichungen:

$$20 \text{ m}^3 \text{ Gülle} * 0,6 \text{ Gew.-\%} = 1,2 \text{ t oTS} \qquad \text{(Gleichung 1)}$$

$$1,2 \text{ t oTS} * 500 = 600 \text{ m}^3 \qquad \text{(Gleichung 2)}$$

eine Biogasausbeute von 600 m³/Tag.

Wird ein Fermenter derselben Größenordnung hingegen mit Energiepflanzen beschickt, so kann aufgrund des höheren oTS-Anteils eine höhere Raumbelastung gefahren werden, was sich in erheblich verbesserten reaktorspezifischen Biogasausbeuten äußert.

Da ein Biogasfermenter einen erheblichen Eigenenergieverbrauch aufweist (insbesondere für das Rührwerk und die Heizung), ist eine ausschließliche oder überwiegende Verwendung von Materialien mit geringem Anteil an organischer Trockensubstanz (oTS), wie z.B. Güllen, in einem herkömmlichen Biogasfermenter nicht wirtschaftlich.

Gleichwohl besteht auf landwirtschaftlichen Betrieben ein erheblicher Bedarf für eine biologische Aufbereitung vonWirtschaftsdüngern (Tierexkrementen und Fäkalien), insbesondere von Gülle.

Die Ausbringung von Gülle auf landwirtschaftliche Flächen unterliegt strengen gesetzlichen Auflagen. So dürfen auf Milchviehweiden grundsätzlich nur hygienisierte Güllen ausgetragen werden. Diese Hygienisierung erfolgt auf chemischem (mit NaOH) oder thermischem Wege und verursacht in jedem Falle erhebliche Kosten.

Zwar wäre eine Hygienisierung in einem Biogasfermenter, der im thermophilen Bereich (> 55 °C) betrieben wird, denkbar, allerdings eignen sich wie oben angedeutet herkömmliche Biogasfermenter nicht für eine effektive großmassstäbliche Aufbereitung von Gülle.

Hinzu kommt, dass sowohl hygienisierte als auch unbehandelte Gülle nach dem Ausbringen auf einen Acker, eine Wiese oder Weide die Entstehung von klimaschädlichen Gasen begünstigt, und zwar nicht nur von Kohlendioxid ($CO_2$), sondern insbesondere auch von Methangas ($CH_4$), Ammoniak ($NH_3$) und Lachgas ($N_2O$), die in die Atmosphäre entweichen und so den Treibhauseffekt begünstigen.

Aufgabe der vorliegenden Erfindung ist es daher, einen Fermenter sowie ein Verfahren zur Erzeugung von Biogas bereitzustellen, der die wirtschaftliche Fermentation von organischem Material mit geringem Anteil an organischer Trockensubstanz (oTS) ermöglicht.

Dieser Fermenter soll aber auch mit hochkonzentrierten Substratmischungen bei hoher Raumbelastung (> 5 kg oTS/m³ Faulraum x d) hocheffizient und stabil Methan erzeugen können. Dies gelingt u.a. auf Grund der fixierten Biomasse und der Rückgewinnung der aktiven Biomasse (vorzugsweise Methanbildner) zur Rückspeisung und Animpfung im Anmischbereich.

Weitere Aufgabe ist es, einen Fermenter sowie ein Verfahren für die wirtschaftliche Aufbereitung und Hygienisierung von Gülle bereitzustellen.

Diese Aufgabe wird mit den Merkmalen des vorliegenden Anspruchs 1 gelöst. Die Unteransprüche geben bevorzugte Ausführungsformen an. Dabei ist zu beachten, dass die genannten Bereichsangaben durchweg einschliesslich der jeweiligen Grenzwerte zu verstehen sind.

Demnach ist ein Verfahren zur Erzeugung von Biogas aus pumpbarem organischen Material mit geringem Anteil an organischer Trockensubstanz in einem Fermenter vorgesehen, wobei das Verfahren die folgenden Schritte aufweist:

i) Einbringen des pumpbaren organischen Materials durch einen Zulauf in den Fermenter,
ii) Erzeugung und Aufrechterhaltung eines anaeroben Milieus, eines pH-Werts von mindestens 7 und einer Temperatur im mesophilen bis thermophilen Bereich,
iii) Erzeugung eines Materialstroms des pumpbaren organischen Materials durch einen Festbettreaktor sowie einen Absetzraum des Fermenters,
iv) Rückgewinnung der spezifisch leichteren Fraktionen des pumpbaren organischen Materials in einem Rückgewinnungsabschnitt,
v) sowie Auffangen des entstehenden Gases und kontinuierliche oder schubweise Entnahme des vergorenen Gärrestes.

Unter der Definition "organisches Material mit geringem Anteil an organischer Trockensubstanz (oTS)" sollen im wesentlichen solche Materialien verstanden werden, die einen oTSAnteil von weniger als 50 Gew.-% aufweisen, bevorzugt weniger als 25 Gew.-% und besonders bevorzugt weniger als 10 Gew.-%.

Ein solches Material stellt z.B. Gülle, also d.h. ein aus Tierexkrementen, Urin, Einstreu, Futterresten und Tränkwasserverlusten bestehendes Wirtschaftsdüngersubstrat dar, das meist einen oTS-Anteil von weniger als 10 Gew.-%- aufweist.

Ein ebensolches Material stellt z.B. auch der Gärrest aus einer konventionellen Rührkessel-Biogasanlage dar, aber auch Stoffe wie Schlempe (Rückstände aus der Alkoholvergärung, insbesondere der Bioethanolproduktion), Klärschlämme oder hochbelastete Abwässer aus der Nahrungsmittelindustrie. Weiterhin fallen unter die obige Definition auch Gemische aus Gülle oder Gärresten mit Substraten aus Nachwachsenden Rohstoffen (NaWaRO).

Durch die Verwendung eines Festbettreaktors werden eine Reihe von Vorteilen erzielt. So macht ein Festbettreaktor ein eigenes Rührwerk verzichtbar, wie dies in Rührkesselfermentern Verwendung findet, da sich innerhalb des Festbettreaktors ein gerichteter Materialstrom einstellen lässt. Dies ist zu vergleichen mit parallel ausgerichteten Darmpaketen, die innen und außen mit Darmzotten besetzt sind und so eine große Besiedlungsfläche für Mikroben bieten und zu einem je nach Verfahrensabschnitt aufwärts oder abwärts gerichteten Substratstrom führen.

Stattdessen kann eine energieeffizientere Pumpe verwendet werden, insbesondere eine Doppelkolbenpumpe. Die herkömmlicherweise verwendeten Rührwerke weisen standardmäßig eine Leistungsaufnahme von etwa 18 kW auf. Durch Verwendung einer Doppelkolbenpumpe sind hier Energieeinsparungen von bis zu 90 % möglich. Auf diese Weise wird die Wirtschaftlichkeit des erfindungsgemäßen Fermenters erheblich erhöht

Durch den gerichteten Materialstrom (Zwangspassage) werden insbesondere auch die in Rührkesselfermentern unvermeidlichen Kurzschlussströme verhindert, die eine effektive Hygienisierung des Gärmaterials sowie eine optimale Vergärung beeinträchtigen. Auf beide Punkte wird weiter unten noch eingegangen.

Hinzu kommt, dass der vorgesehene Festbettreaktor ein Besiedlungssubstrat für methanbildende Mikroorganismen bereitstellt. Auf diese Weise können sich im Unterschied zu einem Rührkesselfermenter geschichtete Mikrobiozönosen einstellen.

Dadurch gelingt es, den zweiten Stoffwechselweg der Methanerzeugung erst in größerem Umfang zu ermöglichen und ihn sogar zu optimieren. Hierbei arbeiten Mikroben auf engem Raum so effizient zusammen, dass $H^+$ und $CO_2$ (als $HCO_3^-$) zu $CH_4$ (Reaktion 2) synthetisiert werden können. Dadurch wird der $CO_2$-Gehalt im Biogas reduziert und der $CH_4$-Gehalt entsprechend gesteigert. Dies dient der Qualitätsverbesserung und Effizienzsteigerung.

Dies ist sehr wichtig, da bestimmte, für die Biogasbildung erforderliche Bakterien und Mikroorganismen keinen Kontakt mit dem zu vergärenden Substrat bekommen dürfen, während andere Mikroorganismen diesen Kontakt brauchen. In einem Rührkesselfermenter, in dem den Mikroorganismen kein Besiedlungssubstrat zur Verfügung gestellt wird, kann sich diese Schichtung nicht ausbilden, was zu erheblich geringerer Biogasausbeute führt.

So unterscheidet man bei der Biogassynthese insbesondere die Schritte der Acetogenese und der Methanogenese, für deren Ablauf jeweils unterschiedliche Mikroorganismen verantwortlich sind. Während der Acetogenese werden die niederen Fett- und Carbonsäuren sowie die niederen Alkohole durch acetogene Mikroorganismen primär zu Essigsäure, bzw. deren gelöstem Salz, dem Acetat umgesetzt. Während der Methanogenese, die obligat anaerob abläuft, wird die Essigsäure durch entsprechend acetoclastische Methanbildner in Methan und Kohlenstoffdioxid sowie Wasserstoff umgewandelt. Die jeweiligen Mikroorganismen stehen miteinander in einer Symbiose, d.h. dass die einen Mikroorganismen die Stoffwechselprodukte der anderen als Substrat bzw. Edukt verwenden können.

Gerade der Stoffwechselweg

$$2) \qquad HCO_3^- + H^+ + 4H_2 \, \text{-}\,\text{-} > CH_4 + 3\,H_2O$$

gelingt bei konventionellen Fermentern kaum, da der H -Ionen-Übergang im Bereich einer Nanosekunde zu erfolgen hat und dies eine Immobilisierung der symbiontischen Mikroben voraussetzt um die dafür nötige räumliche Nähe sicherzustellen.

Hinzu kommt, dass sich im Festbettreaktor die Mikroorganismen schneller adaptieren und durch die Rückgewinnung der aktiven (methanbildenden) Biomasse der Fermenter also schneller "einfährt" (da permanent rückgeimpft wird), und so die erwähnte Propionsäure besser und schneller durch die Mikroorganismen abgebaut wird, bzw. erst garnicht entstehen kann.

Erfindungsgemäss ist ferner ein Fermenter geeignet zur Durchführung des Verfahrens zur Erzeugung von Biogas aus pumpbarem organischen Material mit geringem Anteil an organischer Trockensubstanz vorgesehen, aufweisend

a) mindestens einen Zulauf für das pumpbare organische Material,
b) mindestens einen Festbettreaktor für das pumpbare organische Material mit mindestens einem primären und einem sekundären Abschnitt, sowie
c) mindestens einen Ablauf für den entstehenden Gärrest,

wobei dieser außerdem

d) mindestens einen Absetzraum für das pumpbare organische Material aufweist, der zwischen dem primären und sekundären Abschnitt des Festbettreaktors angeordnet ist, sowie

e) mindestens einen Rückgewinnungsabschnitt, der mit dem Absetzraum in Verbindung steht und so ausgebildet ist, dass spezifisch leichtere Fraktionen des pumpbaren organischen Materials zurückgewonnen werden können.

Der Absetzraum kann bei zwei getrennten Verfahrensteilen in jedem Kopfteil zu finden sein.

Durch den erstmals vorgesehenen Rückgewinnungsabschnitt werden spezifisch leichtere Fraktionen des pumpbaren organischen Materials zurückgewonnen und können dem aufsteigenden (primären) Abschnitt des Festbettreaktors erneut zugeführt werden.

Bei diesen spezifisch leichteren Fraktionen handelt es sich einerseits um spezifisch leichtere organische Fraktionen, wie z.B. flüchtige Fettsäuren oder faserige Biomasse, in der sich die Methanbildner und ihr gebildetes Gas auf Grund des durch die Methan- und Kohlendioxidfreisetzung bedingten Auftriebs verfangen. In herkömmlichen Rührkesselfermentern bilden diese Fraktionen eine Schwimmschicht aus und entziehen sich auf diese Weise dem Vergärungsprozess, bzw. bergen das Risiko eines sich aufbauenden Substratüberdrucks, wenn sich die Mikrogasbläschen nicht mehr aus der Schwimmschicht lösen können, etwa beim Ausfall des Rühraggregates).

Die flüchtigen Fettsäuren gehen im Übrigen z.T. leicht in die Gasphase über und werden dadurch dem weiteren Vergärungsprozess dauerhaft entzogen.

Weiterhin sind in diesen spezifisch leichteren organischen Fraktionen Mikroorganismen (sog. "aktive Biomasse") enthalten, die sich vom Substrat des Festbettreaktors gelöst haben und ohne einen Rückgewinnungsabschnitt mit dem Gärrest aus dem Fermenter ausgetragen werden würden. Dies bedingt auch einen stetigen Verlust an Spurenelementen, die in konventionellen Rührkesselfermentern supplementiert werden müssen. Dies verursacht zusätzliche Kosten und bedeutet einen zusätzlichen Eintrag von Schwermetallen. Hierdurch wird in herkömmlichen Biogasfermentern die Besatzdichte an Biogas erzeugenden Mikroorganismen stetig reduziert, so dass diese Fermenter grundsätzlich mit einer für eine optimale Biogasausbeute zu geringen Mikroorganismendichte betrieben werden. Der erfindungsgemäße Rückgewinnungsabschnitt erlaubt die Rückgewinnung und erneute Einspeisung dieser Mikroorganismen in den Fermenter, so dass der erfindungsgemäße Fermenter eine erheblich höhere Mikroorganismendichte aufweist als ein herkömmlicher Fermenter und zusätzlich eine wesentlich bessere Versorgung mit Spurenelementen, die über die aktive Biomasse im Kreislauf geführt werden.

Es bietet sich so die Möglichkeit, die aktive Biomasse, die in Vorrichtungen nach dem Stand der Technik zusammen mit dem ausgegorenen Material in ein Gärrückstandslager verbracht wird und dort ungenutzt verbleibt, zurückzugewinnen und dem Gärprozess erneut zuzuführen. Auf diese Weise wird der Ertrag in den konventionellen Fermentern wesentlich erhöht. Hinzu kommt, dass durch die Rückführung der aktiven Biomasse in den Gärraum der Zeitraum zur Optimierung der Anlage im Erstbetrieb wesentlich verkürzt wird. Grundsätzlich ist es so, dass eine Biogasanlage eine gewisse Zeit zur Optimierung braucht. Ursache hierfür ist, dass sich in der Anlage zunächst eine stabile Mikroorganismen-Flora einstellen muss. Durch die Möglichkeit, die in dem aus dem Gärraum entnommenen, durchgegorenen Material noch befindlichen Mikroorganismen zurück zu gewinnen, wird der Zeitraum bis zum Aufbau einer stabilen und hocheffizienten Mikroflora erheblich verkürzt. Auf diese Weise wird der maximale Ertrag also wesentlich schneller erreicht.

Die Möglichkeit der Rückführung der aktiven Biomasse weist noch einen anderen Vorteil auf: Da die Besatzdichte an aktiven Mikroorganismen im Gärraum auf einem wesentlich höheren Niveau gehalten werden kann, wird der Gärprozess beschleunigt. Daher kann der Durchsatz des Fermenters erhöht werden. So kann ein erfindungsgemäßer Fermenter wesentlich höhere Raumbelastungen tolerieren.

Dieser Effekt stellt sich auch bei der Kombination des erfindungsgemäßen Fermenters mit einem konventionellen Rührkesselfermenter ein, wenn die rückgewonnene aktive Biomasse in den Rührkesselfermenter zurückgeführt wird (sogenanntes "Repowering", siehe unten).

Hinzu kommt, dass der erfindungsgemäße Fermenter die in herkömmlichen Rührkesselfermentern oftmals erforderliche Zugabe von Spurenelementen entbehrlich macht, da auch die in der aktiven Biomasse enthaltenen Spurenelemente durch die Biomasserückführung zurückgeführt werden.

Weiterhin muß in herkömmlichen Rührkesselfermentern der Fermenterinhalt stets wirksam durchmischt werden. Durch den Rührvorgang werden die Symbiosen der verschiedenen Mikroorganismen, insbesondere der Methanbakterien, permanent gestört. Eine länger dauernde Prozessstabilität kann so auf hohem Leistungsniveau nicht erreicht werden. Das Faulsubstrat muss hier den Mikroorganismen gleichzeitig als Nährsubstrat und als Besiedlungsfläche ihrer Symbiosekolonien dienen. Das Substrat muss daher eine gewisse Mindeststruktur bieten. Im erfindungsgemäßen Fermenter bilden sich dagegen äußerst stabile Symbiosen fest angesiedelter Methanbakterien, wodurch die Methanisierung auch bei leicht abbaubaren Substraten mit geringer Strukturalität optimal verlaufen kann.

Bakterien, die freien Wasserstoff produzieren und solche, die den Wasserstoff verwerten, sollten für eine optimale Symbiose fest angesiedelt sein. Daher kann im erfindungsgemäßen Fermenter auf den Besiedlungsflächen der Festbettreaktoren optimal Methangas produziert werden.

In den restlichen Bereichen des erfindungsgemäßen Fermenters entfaltet sich die Biologie optimal und wird insbesondere nicht durch Aufrührvorgänge gestört.

Die Mikroorganismen benötigen zur optimalen Fermentation nicht nur organische Säuren, sondern auch $CO_2$.

Im Bereich des Zulaufs für das organische Material bildet sich verstärkt $CO_2$, welches in optimaler Weise den darüber liegenden aufsteigenden Abschnitt des Festbettreaktors durchströmt. Dieses $CO_2$ steht somit den Methanbakterien ausreichend zur Methanisierung zur Verfügung. Somit wird eine optimale biologische Stabilität erreicht.

Der erfindungsgemäße Fermenter weist daher eine verbesserte Biogasausbeute, insbesondere bei Materialien mit geringem oTS-Anteil auf. Die verbesserte Ausbeute resultiert aus einem weitergehenden Abbau der oTS, insbesondere durch besseren Abbau der Essigsäure-Äquivalente im Ablauf der Anlage sowie durch erneute Nutzung der wiedergewonnenen Bakterien, und zwar durch Ausnutzung von deren Stoffwechselaktivitäten und/oder, falls abgestorben, durch Ausnutzung von deren Biomasse.

Insgesamt weist der erfindungsgemäße Fermenter noch weitere Vorteile auf:

- Die Verweildauer der Substrate (insbesondere Gülle) wird von zuvor 50 auf 10 Tage reduziert; hierdurch erhöht sich der Durchsatz.

- Der Wärmebedarf wird rechnerisch auf 20 % reduziert (in der Praxis reicht in der Regel bereits die intrinsische Prozesswärme, d.h. eine externe Wärmezufuhr ist verzichtbar).

- Das Fermentervolumen kann im Vergleich zu einem herkömmlichen Rührkesselfermenter von 1000 m$^3$ auf 200 m$^3$ reduziert werden, was die Bau- und Investitionskosten reduziert.

In einer bevorzugten Ausführungsform des erfindungsgemäßen Fermenters ist vorgesehen, dass der primäre Abschnitt des Festbettreaktors ein aufsteigender Abschnitt und der sekundäre Abschnitt des Festbettreaktors ein absteigender Abschnitt ist.

Auf diese gelingt es, den zweiten Stoffwechselweg der Methanerzeugung erstmals in größerem Umfang zu ermöglichen und zu optimieren. Hierbei arbeiten Mikroben auf engem Raum so effizient zusammen, dass $H^+$ und $HCO_3^-$ zu $CH_4$ (siehe Reaktion 2) synthetisiert werden können. Dadurch wird der $CO_2$-Gehalt im Biogas reduziert und der $CH_4$-Gehalt entsprechend gesteigert. Dies dient der Qualitätsverbesserung und Effizienzsteigerung. Im aufsteigenden Bereich besteht durch die fein verteilten $CO_2$-Bläschen und deren große spezifische Oberfläche, die Chance, dass die Methanbildner in intensivem Kontakt diesen zweiten und wesentlich schwieriger zu realisierenden Pfad der Methanbildung beschreiten können.

Der erfindungsgemäße Fermenter ist jedoch auch in der Lage, mit hochkonzentrierten Substratmischungen bei hoher Raumbelastung (> 5 kg oTS/m$^3$ Faulraum x d) hocheffizient und stabil Biogas erzeugen. Dies gelingt auf Grund der fixierten Biomasse und der Rückgewinnung der aktiven Biomasse (vorzugsweise Methanbildner) zur Rückspeisung und Animpfung im Anmischbereich. Bei der Kombination der getrennten Abschnitte der Vergärung mit Aufwärtsströmung (Upflow) und Abwärtsströmung (Downflow) wird dabei überdies die Dünn- und Dickdarmfunktion simuliert und damit Prinzipien der Bionik in die Verfahrenstechnik übertragen.

Eine vorgeschaltete Langzeithydrolyse bzw. der Anmischbereich übernehmen in dieser Analogie gleichsam die Magenfunktion, durch die eine effiziente Versauerung des Inputsubstrates erfolgt.

Zusätzlich kann $CO_2$ aus externen Quellen zur Intensivierung der Reaktionen in den aufsteigenden Abschnitt eingespeist werden. Letzteres kann aus externen Quellen stammen, insbesondere aber auch aus einem vorgeschalteten Langzeit-Hydrolysereaktor.

Weiterhin ist bevorzugt vorgesehen, dass der Festbettreaktor ein Material aufweist, das eine große Besiedlungsoberfläche für Mikroorganismen zur Verfügung stellt

Hier kommen z.B. Materialien mit einer strukturierten Oberfläche und/oder einer inneren Oberfläche in Frage. Dies können z.B. Materialien mit einer strukturierten Kunststoffoberfläche sein, aber auch Lavagranulat, Blähton, Keramikpellets, textile, metallene oder hölzerne Strukturen und dergleichen.

Auf diese Weise wird eine große Besiedlungsoberfläche und damit eine hohe Besatzdichte und Stabilität für die Biogas erzeugenden symbiontisch lebenden Mikroorganismenkolonien ermöglicht.

Auf diese Weise wird eine große Besiedlungsoberfläche und damit eine hohe Besatzdichte und Stabilität für die Biogas erzeugenden symbiontisch lebenden Mikroorganismen ermöglicht.

Besonders bevorzugt ist vorgesehen, dass der Festbettreaktor ein Material aufweist, das die Ausbildung von im wesentlichen longitudinal angeordneten Kanälen ermöglicht.

Unter dem Begriff "im wesentlichen longitudinal angeordneten Kanälen" sollen solche Materialien verstanden werden, die geeignet sind, dem zu fermentierenden Substrat im aufsteigenden (primären) und/oder absteigenden (sekundären) Abschnitt des Fermenter eine einheitliche Fließrichtung zu geben. Dies ist überdies auch vorteilhaft, um Kurzschlussströme zu verhindern. Hierauf wird weiter unten noch eingegangen. Mögliche Materialien für diesen Zweck sind z.B. senkrecht angeordnete Röhren aus Keramik, Ton, Steingut, Metall, Holz oder Kunststoff, oder senkrecht angeordnete Stäbe, Bretter, Waben, Seile, Leinen oder Schnüre.

Besonders bevorzugt weist der Festbettreaktor dabei ein Material auf, das sowohl eine große Besiedlungsoberfläche für Mikroorganismen zur Verfügung stellt als auch die Ausbildung von im wesentlichen longitudinal angeordneten Kanälen ermöglicht.

Hier ist insbesondere an Kunststoffrohre mit einer vergrößerten Oberfläche, wie z.B. die bekannten flexiblen Tiefbau-Drainagerohre mit Durchmessern zwischen 50 und 400 mm gedacht. Diese weisen eine gewellte Wandstruktur auf, die es ermöglicht, dass sowohl die Außen- als auch die Innenfläche dieser Rohre von Mikroorganismen besiedelt werden kann.

Die besagten Rohre sind besonders deswegen vorteilhaft, weil sie insbesondere im aufsteigenden Abschnitt des Festbettreaktors dafür sorgen, dass die aufsteigenden Gasblasen (insbesondere $CO_2$) eine bestimmte Größe nicht überschreiten. In herkömmlichen Fermentern wachsen die aufsteigenden Gasblasen durch Abnahme des hydrostatischen Drucks sowie durch Aufnahme weiterer Gasblasen überproportional stark an, was einerseits ihre relative Oberfläche verringert und andererseits ihre Aufstiegsgeschwindigkeit stark zunehmen lässt. Beides ist Ursache dafür, dass das aufsteigende $CO_2$ praktisch nicht mehr verstoffwechselt und so nicht mehr gemäß Reaktion 2) in Methangas umgewandelt werden kann. Die Ausführung mit Rohren oder ähnlichen Hohlkörperstrukturen setzt der Größenzunahme der Blasen Grenzen und sorgt durch die Kompartimentierung des Substratstroms so dafür, dass aufsteigendes $CO_2$ in parallelen und dadurch stabilisierten Strukturen weiter verstoffwechselt werden kann.

Hierbei ist bevorzugt vorgesehen, dass am jeweils oberen und unteren Ende des Festbettreaktors eine Halteeinrichtung für diese Kunststoffrohre angeordnet ist, welche den bestmöglichen Abstand der Rohre zueinander fixiert und die Rohrdurchgänge nicht verengt, bzw. solchen Verengungen vorbeugt.

Diese Halteeinrichtung kann z.B. aus Edelstahlrohrabschnitten ("Manschetten") bestehen, die in einer Fläche angeordnet und über Winkel miteinander verschweißt, gesteckt, verschraubt oder vernietet sind.

Bevorzugt werden dabei Kunststoffrohre mit einem Innendurchmesser von 100 - 300 mm und einem Abstand zueinander von 50 - 300 mm verwendet. Besonders bevorzugt beträgt der Innendurchmesser 200 mm und der Abstand der Rohre zueinander 100 mm.

Durch die Anordnung aus mindestens einem Festbettreaktor mit mindestens einem aufsteigenden (primären) und einem absteigenden (sekundären) Abschnitt wird insbesondere die Ausbildung von Kurzschlußströmen verhindert. Dies ist insbesondere deswegen wichtig, weil nur durch eine solche Zwangspassage gewährleistet werden kann, dass das Material bestmöglich vergoren (d.h. mineralisiert) wird, und andererseits eine vollkommene Hygienisierung des Gärmaterials möglich wird.

Letztere ist für Materialien, die Tierexkremente enthalten oder aus solchen hergestellt wurden, vor der Ausbringung auf bestimmte landwirtschaftlich genutzte Flächen, wie z.B. Milchviehweiden, aufgrund gesetzlicher Bestimmungen erforderlich. Dasselbe gilt für die Ausbringung in Wasserschutzgebieten.

Die erfindungsgemäße Anordnung stellt sicher, dass das gesamte zu vergärende Material den gesamten Festbettreaktor passiert. Wird dieser im thermophilen Temperaturbereich (d.h. bei Temperaturen von mehr als 55 °C, reicht bereits eine Verweildauer von 24 Stunden für eine ausreichende Hygienisierung aus.

Die Hygienisierung inaktiviert dabei mesophile Keime (pathogene, fakultativ pathogene und nicht pathogene), wie z.B. coliforme Bakterien, Salmonellen, Brucelloseerreger und dergleichen mehr. Die für die Biogassynthese notwendigen Mikroorganismen sind durchweg thermophil, so dass Sie die besagten Temperaturen nicht nur unbeschadet überstehen, sondern dort auch ihr Aktivitätsmaximum entfalten. Hinzu kommt, dass Sie aufgrund des guten Besiedlungssubtrats im Fermenter verbleiben und nicht mit dem Gärrest ausgespült werden, also auch nicht z.B. auf eine Milchviehweide ausgebracht werden.

Unter bestimmten Voraussetzungen reicht schon die bei der Biogassynthese freiwerdende Wärme aus, dass sich thermophile Bedingungen im Fermenter einstellen, d.h. eine externe Wärmezufuhr ist verzichtbar, was zu erheblichen Energieeinsparungen führt.

Der erfindungsgemäß vorgesehene Rückgewinnungsabschnitt, der in einer besonders bevorzugten Ausgestaltung über eine Überlaufkante mit dem Absetzraum in Verbindung steht, ist so ausgebildet, dass spezifisch leichtere Fraktionen des pumpbaren organischen Materials zurückgewonnen werden und dem aufsteigenden (primären) Abschnitt des Festbettreaktors erneut zugeführt werden können. Diese spezifisch leichteren Fraktionen enthalten insbesondere einen großen Teil der methanogenen Mikroorganismen, die ansonsten aus dem Fermenter ausgespült würden und für die Fermentation verloren gehen würden.

Dieser Effekt wird dadurch unterstützt, dass sich die besagten methanogenen Mikroorganismen auf den Oberflächen des Festbetts ansiedeln. So können sie nicht ausgeschwemmt werden.

Alternativ kann dieses rückgewonnene aktive Material auch im Rahmen des "Repowering" in den zu intensivierenden konventionellen Fermenter zurückgeführt werden, um dort die Konzentration an Methanbildnern anzuheben und eine Steigerung des Durchsatzes bzw. der Leistung zu ermöglichen.

Ebenso kann vorgesehen sein, dass der Rückgewinnungsabschnitt über periphere Bohrungen bzw. Siebeinrichtungen mit dem Absetzraum in Verbindung steht. Für den Fachmann ergeben sich jedoch ohne Zutun erfinderischer Tätigkeit aus dieser Lehre auch andere Möglichkeiten, wie die oben angesprochene Verbindung zwischen Rückgewinnungsabschnitt und Absetzraum bewerkstelligt werden kann.

Darüber hinaus kann vorgesehen sein, dass an der Überlaufkante den Bohrungen bzw. Siebeinrichtungen ein Abstreifer vorgesehen ist, der eine Verstopfung derselben bzw. die Ausbildung einer Schwimmschicht an der Überlaufkante verhindert.

Das rückgewonnene Material, das in einigen Ausführungsformen der vorliegenden Erfindung auch als "Impfschlamm" bezeichnet wird, kann dem dem Fermenter neu eingespeisten, zu vergärenden organischen Material zugeführt werden. Hierfür ist bevorzugt eine Dosiereinrichtung vorgesehen, die bevorzugt elektronisch oder mit einem Mikroprozessor gesteuert wird. Auf diese Weise wird die Konzentration der methanbildenden Mikroorganismen dauerhaft erhöht, was wiederum der Biogasausbeute und -qualität zugute kommt.

Grundsätzlich kann durch die Ausgestaltung des Rückgewinnungsabschnitts das Volumenverhältnis zwischen dem dem Fermenter neu eingespeisten Material und dem im Rückgewinnungsabschnitt zurückgehaltenen Material eingestellt werden. Dies kann z.B. durch die gezielte Auswahl der Höhe einer vorgesehenen Überlaufkante gegenüber dem oberen Ende des aufsteigenden Abschnitts des Festbettreaktors erfolgen. Ebenso kann dies z.B. über die gezielte Auswahl der Größe und/oder Dichte der peripheren Bohrungen erfolgen. Für den Fachmann ergeben sich jedoch ohne zutun erfinderischer Tätigkeit aus dieser Lehre auch andere Möglichkeiten, wie die oben angesprochene Einstellung des Volumenverhältnisses bewerkstelligt werden kann.

Dabei ist bevorzugt vorgesehen, dass das Volumenverhältnis zwischen dem dem Fermenter neu eingespeisten Material und dem im Rückgewinnungsabschnitt zurückgehaltenen Material im Bereich zwischen 1 : 0,9 - 2 : 0,1 liegt. Besonders bevorzugt beträgt das Volumenverhältnis 2 : 1. Die "Rückimpfung" mit dem zurückgehaltenen Material soll in jedem Falle so groß sein, dass eine problemlose Fermentation gewährleistet ist und eine partielle Übersäuerung nicht stattfindet. Dies lässt sich mit geeigneten Methoden (pH-Meter, NIRS, GC-Beprobung) für den Fachmann leicht nachvollziehen.

Weiterhin ist bevorzugt vorgesehen, dass der Rückgewinnungsabschnitt aus einem ggf. mehrteiligen, im wesentlichen senkrecht angeordneten röhrenförmigen Element besteht.

Der Rückgewinnungsabschnitt ist überdies bevorzugt zwischen dem aufsteigenden (primären) und dem absteigenden (sekundären) Abschnitt mindestens eines Festbettreaktors angeordnet.

Diese Ausführungsform weist eine Reihe von Vorteilen auf. So kann das im Rückgewinnungsabschnitt noch erzeugte Biogas von derselben Gasauffangeinrichtung aufgefangen werden, die auch das in den Abschnitten des oder der Festbettreaktoren erzeugte Gas auffängt. Hinzu kommt, dass auf diese Weise der Rückgewinnungsabschnitt leicht auf dieselbe Temperatur zu bringen ist wie die der oder die Festbettreaktoren. Weiterhin weist der Rückgewinnungsabschnitt so eine optimale Position für die Rückgewinnung der spezifisch leichteren Fraktionen auf, da er auf diese Weise in der Mitte des Absetzraums angeordnet ist; insbesondere dann., wenn der obere Rand des Rückgewinnungsabschnitts eine Überlaufkante bildet. Hinzu kommen im übrigen fertigungstechnische Vorteile, auf die weiter unten noch eingegangen wird.

Gleichwohl ist es jedoch nicht ausgeschlossen und daher vom Schutzumfang der vorliegenden Ansprüche sehr wohl erfasst, dass der Rückgewinnungsabschnitt nicht zwischen dem aufsteigenden (primären) und dem absteigenden (sekundären) Abschnitt mindestens eines Festbettreaktors angeordnet ist, sondern z.B. seitlich oder außerhalb des eigentlichen Fermenters.

In einer besonders bevorzugten Ausführungsform ist ein weiterer Rückgewinnungsabschnitt nach dem absteigenden (sekundären) Abschnitt des Festbettreaktors angeordnet. Auf diese Weise wird die Rückgewinnung der genannten Substrate und Mikroorganismen nochmals verbessert.

Der Fermenter weist bevorzugt die äußere Form eines oder zweier stehend angeordneten Zylinder(s) auf. Dabei kann vorgesehen sein, dass der Fermenter bzw. der/die Zylinder aus mehreren Segmenten besteht/bestehen, die in einem Fertigungsbetrieb herstellbar sind und an Ort und Stelle zu einem Fermenter zusammenfügbar sind.

Hier können z.B. zwei Zylinderhälften oder mehrere Zylinderabschnitte vorgesehen sein, die an Ort und Stelle aufgerichtet und miteinander verschweißt oder über Befestigungswinkel miteinander verschraubt werden. Idealerweise weist eine der Zylinderhälften bzw. der Zylinderabschnitte bereits den Rückgewinnungsabschnitt auf, was darüber hinaus die Fertigung und Montage erleichtert und so Kosten reduziert.

Die Erfinder haben errechnet, dass sich ein solchermaßen vorgefertigter Fermenter mit einem Volumen von 200 - 250 m$^3$ innerhalb von ein bis zwei Tagen an Ort und Stelle errichten lässt. Auf diese Weise wird der Montageaufwand (Arbeitsstunden, Gerät, Mobilkran) und die damit verbundenen Kosten erheblich reduziert. Überdies werden die Betriebsabläufe an Ort und Stelle (z.B. auf einem landwirtschaftlichen Betrieb) geringstmöglich beeinträchtigt. Hinzu kommt, dass auf diese Weise eine standardisierte Herstellungsweise bewerkstelligt und so der Qualitätsstandard des Fermenters besser gewährleistet werden kann.

Weiterhin kann vorgesehen sein, dass der Fermenter eine zumindest teilweise oberhalb des Festbettreaktors und/oder der Rückgewinnungseinrichtung angeordnete Gasauffangeinrichtung aufweist.

Bei dieser Gasauffangeinrichtung kann es sich z.B. um eine an sich bekannte Kuppel bzw. Dachkonstruktion mit einer darunter angeordneten gasdichten Membran handeln. In einer solchen Ausgestaltung kann insbesondere vorgesehen sein, dass die Gasauffangeinrichtung auch als Gasspeichereinrichtung fungiert. Dabei hängt die gasdichte Membran, solange sich noch nicht viel Gas entwickelt hat, relativ schlaff über dem Gärraum, wird dann aber durch das entstehende Gas nach oben verdrängt und gespannt. Das gebildete Gas kann dann auf bekannte Weise und mit bekannten Entnahmevorrichtungen entnommen werden.

Grundsätzlich kann vorgesehen sein, dass der erfindungsgemäße Fermenter außerdem eine Einrichtung zur Einspeisung des erzeugten Biogases in ein Gasleitungsnetz aufweist.

Es ist allerdings bevorzugt vorgesehen, dass der erfindungsgemäße Fermenter mit einer Vorrichtung zur Verstromung des erzeugten Biogases gekoppelt ist.

Um die in dem erzeugten Biogas gebundene chemische Energie in elektrische Energie umzuwandeln, wird das Biogas beispielsweise in einem Blockheizkraftwerk (BHKW) mit darin enthaltenem Gasmotor oder Zündstrahlmotor verstromt. Um wirtschaftlich arbeiten zu können, muss das zu verbrennende Gas dem Gasmotor mit einem Vordruck von etwa 100 mbar geliefert werden. Bei herkömmlichen Biogasanlagen ist hier ein eigenes Gasdruckgebläse erforderlich, um das gespeicherte Gas auf den genannten Vordruck zu bringen. Dieses Gebläse verbraucht einerseits nicht unerhebliche Mengen an Energie, andererseits erhöht es den Wartungsaufwand und die Anschaffungskosten sowie den Steuerungsaufwand einer Biogasanlage.

Besonders bevorzugt ist vorgesehen, dass der Fermenter eine hydrostatische Gasspeichereinrichtung aufweist.

Unter dem Begriff "hydrostatische Gasspeichereinrichtung" soll im Folgenden eine Gasspeichereinrichtung verstanden werden, in welcher das eingeleitete Gas eine zuvor vorhandene Flüssigkeit (insbesondere Wasser) gegen die Schwerkraft (und damit gegen einen sich aufbauenden hydrostatischen Druck bzw. eine Wassersäule) verdrängt. In Bezug auf diese Ausführungsform wird auf die Zeichnungen verwiesen.

Ist die Gasspeichereinrichtung z.B. so konstruiert, dass das einströmende Gas bei dem Verdrängen der darin befindlichen Flüssigkeit eine maximale Wassersäule von 2000 mm aufbaut, so entspricht dies einem hydrostatischen Druck von 200 mbar. Gleichzeitig wird das gespeicherte Gas auf einem diesem Druck entsprechenden Gasdruck gehalten, und kann unter Verzicht auf ein eigenes Gasdruckgebläse dem Gasmotor des BHKW zugeführt werden. Entscheidend hierfür ist, dass die Biogas erzeugenden Mikroorganismen auch gegen starke Druckgradienten weiterhin Biogas erzeugen können. In der Literatur sind hier Druckgradienten bis zu 160 bar beschrieben. Der beschriebene, sich aufbauende Druckgradient von 200 mbar, der sich u.U. bis in den Fermenter fortsetzt, beeinträchtigt die Biogassynthese also nicht.

Bevorzugt sind die Rohrleitungen zu dem hydrostatischen Gasspeicher so dimensioniert, dass sie die Anforderungen an Gassicherheitseinrichtungen (Über- und Unterdruck) erfüllen. So kann im Überschuß produziertes Gas durch den hydrostatischen Gasspeicher in die Umwelt entweichen, wobei die Flüssigkeit des Gasspeichers als Rückschlagsicherung fungiert und einen Funken- oder Flammeintrag in den Fermenter ausschließt. Eine herkömmliche Gasspeichereinrichtung ist hierzu nicht in der Lage. Überdies können bei entsprechender Dimensionierung der Rohrleitungen diese auch als Überlaufsicherung für übermäßig in den Fermenter eingespeistes Gärsubstrat dienen. Dieses läuft durch die Rohrleitungen ab und wird durch den hydrostatischen Gasspeicher aufgefangen.

Die Gasauffangeinrichtung des Fermenters weist bevorzugt eine kegelförmige, kegelstumpfförmige, paraboloide oder hemispärische Kuppel auf.

Besonders bevorzugt ist diese Kuppel so auf dem Fermenter angeordnet, dass der Bereich der nach oben gerichteten Verjüngung der Kuppel bereits unterhalb einer Überlaufkante des Rückgewinnungsabschnitts beginnt. Es wird in diesem Zusammenhang auf die Zeichnungen verwiesen. Auf diese Weise wird die Rückgewinnung der aktiven Biomasse erheblich verbessert.

Bevorzugt ist weiterhin vorgesehen, dass im Bereich des Gärraums, des Gasspeichers und/oder des Beruhigungsraums keine elektrischen Einrichtungen vorgesehen sind. Ebenso kann der Gärraum, der Gasspeicher und/oder der Beruhigungsraum als Faradayscher Käfig ausgeführt sein. Beide Maßnahmen dienen der Feuer- und Explosionsverhütung. Dazu kann das Gehäuse des Fermenters als Ganzes aus einem leitenden Metall sein (insbesondere $V_4A$-Stahl oder korrosionsfest beschichteter Stahl), oder aber aus einem nichtmetallischen Material, dem ein Netz aus metallischen Leitern beigegeben ist, z.B. in Form eines das Gehäusematerial umgebenden Maschendrahtmaterials.

In einer weiteren bevorzugten Ausgestaltung weist der erfindungsgemäße Fermenter eine im Bodenbereich des Gärraums angeordnete Absetzrinne auf. In dieser kann sich anorganisches Material wie Sand, Kalk, Steine etc. absetzen und, beispielsweise mit Hilfe einer Förderschnecke, aus dem Fermenter entfernt werden. Üblicherweise werden täglich etwa 1 - 3% des Gärmaterials auf diese Weise entfernt. Feststoffe können dann aus dem ausgetragenen Material abgeschieden und die flüssigen Bestandteile wieder in den Gärraum zurückgeführt werden.

In einer weiteren, bevorzugten Ausführungsform ist vorgesehen, dass der Fermenter im Bereich seines Ablaufs einen Wärmetauscher aufweist, mit welchem das frische zu fermentierende organische Material vorwärmbar ist.

Auf diese Weise wird die Einstellung meso- oder thermophiler Bedingungen im Fermenter erheblich erleichtert und gleichzeitig der hierfür erforderliche Energieaufwand reduziert. Im Idealfall reicht die intrinsische, sich bei der Vergärung entwickelnde Reaktionswärme zur Einstellung der genannten Bedingungen aus, so dass eine Wärmezufuhr von außen überflüssig ist.

In einigen Fällen, nämlich dann, wenn die intrinsische Reaktionswärme nicht ausreicht, muss der erfindungsgemäße Biogasfermenter temperiert werden. Viele Heizeinrichtungen, z.B. im Fermenter angeordnete Wärmetauscher, weisen an ihrer Oberfläche eine höhere Temperatur auf, als für die Mikroorganismen optimal ist. Gärmaterial, das mit der Heizeinrichtung in Berührung kommt, wird daher zunächst auf eine oberhalb des bevorzugten Temperaturbereichs liegende Temperatur erwärmt, und gibt diese dann sukzessive an das umgebende Material weiter. Auf diese Weise lässt sich zwar im gesamten Gärraum die gewünschte Temperatur einstellen, allerdings führt die erhöhte Temperatur im Bereich der Heizeinrichtung zum Absterben der dort angesiedelten oder damit in Kontakt gekommenen Mikroorganismen, insbesondere methanogenen Bakterien, und damit zu einer Verminderung des Ertrags.

Weiterhin ist bevorzugt vorgesehen, dass der Fermenter eine Temperierungseinrichtung für das zu vergärende organische Material aufweist, die so eingerichtet ist, dass die Temperatur des Gärmaterials im Gärraum allein durch die Temperierung des über den Zulauf eingebrachten, zu vergärenden organischen Materials einstellbar ist.

Hierfür ist neben einer Heizeinrichtung für das zu vergärende Substrat mindestens ein Temperaturfühler im Gärraum und ein entsprechender Regelkreis erforderlich. Diese Art der Temperierung ist besonders effektiv, da das in den Gärraum eingebrachte, temperierte Material sich sofort verteilt und seine Wärmeenergie schnell an die Umgebung abgibt. Aufgrund des schnellen Wärmetauschs an das umgebende Material werden die Methanbakterien im Fermenter in ihren Lebensvorgängen nicht beeinträchtigt. Außerdem reicht aufgrund der guten thermischen Leitfähigkeit und der effektiven Durchmischung bereits eine geringfügig höhere Temperatur des zu vergärenden Substrats aus, den Fermenter effektiv zu temperieren, sodass auch aus diesem Grunde keine Beeinträchtigung der Methanbakterien im Fermenter zu befürchten ist. Insgesamt wird zudem eine schnellere und gleichmäßigere Temperierung des Gärmaterials ermöglicht, was der Prozessstabilität zugute kommt. Dabei kann bevorzugt vorgesehen sein, dass die Einfülleinrichtung zwischen den beiden Rührwerken angeordnet ist. Das temperierte, zu vergärende Substrat wird auf diese Weise besonders effektiv in den Gärraum eingebracht und vermischt sich schnell mit dem Gärmaterial, wobei es seine Temperatur besonders schnell an die Umgebung abgibt. Außerdem eröffnet sich so die Möglichkeit, das zu vergärende Substrat vor Einbringung in den Gärraum zu pasteurisieren bzw. zu sterilisieren. Auf diese Weise kann es nach Einbringung in den Gärraum besonders schnell von Methanbakterien besiedelt werden, was zu einer Forcierung der Gärung und damit zu einer Steigerung des Ertrags führt. Diese Art der Temperierung erübrigt darüber hinaus das Vorhandensein weiterer Heizeinrichtungen oder Wärmetauscher im Gärraum und verhindert somit die oben genannten negativen Auswirkungen. Ausserdem erübrigt diese Art der Temperierung das Vorhandensein von elektrischen Schaltungen im Gärraum, die ansonsten zu Funken- und damit Explosionsgefahr führen könnten.

Weiterhin ist erfindungsgemäß ein Verfahren zur Erzeugung von Biogas aus pumpbarem organischen Material mit geringem Anteil an organischer Trockensubstanz (oTS) in einem Fermenter gemäß einem der vorherigen Ansprüche vorgesehen. Dieses Verfahren weist die folgenden Schritte auf:

  a) Einbringen des pumpbaren organischen Materials durch einen Zulauf in den Fermenter,
  b) Erzeugung und Aufrechterhaltung eines anaeroben Milieus, eines pH-Werts von mindestens 7 und einer Temperatur im mesophilen bis thermophilen Bereich,
  c) Erzeugung eines Materialstroms des pumpbaren organischen Materials durch den Festbettreaktor sowie den Absetzraum des Fermenters,
  d) Rückgewinnung der spezifisch leichteren Fraktionen des pumpbaren organischen Materials im Rückgewinnungsabschnitt
  e) ggf. erneute Zuführung des rückgewonnenen Materials zu dem Fermenter
  f) Auffangen des entstehenden Gases in und kontinuierliche oder schubweise Entnahme des vergorenen Gärrestes.

Der pH-Wert kann dabei mit den herkömmlichen, dem Fachmann bekannten Mitteln eingestellt werden.

Insbesondere kann überdies vorgesehen sein, dass der Materialstrom durch den Festbettreaktor kontinuierlich oder aber pulsierend erzeugt wird. Beide Varianten können Vor- und Nachteile haben, insbesondere in Bezug auf das jeweils verwendete Substrat. So kann ein pulsierender Materialstrom vorteilhaft sein, um eine längere Kontaktzeit zwischen zu vergärendem Substrat und den Mikroorganismen zu schaffen. Das Einstellen geeigneter Durchflussbedingungen (Geschwindigkeit, Pulsintervalle etc., insbesondere in Bezug auf das jeweils verwendete Substrat) kann der Fachmann durch routinemäßiges Austesten ohne Zutun eigener erfinderischer Tätigkeit leicht selbst in Erfahrung bringen.

Weiterhin ist erfindungsgemäß vorgesehen, dass das rückgewonnene Material mit frischem, zu vergärenden Material vorinkubiert wird, bevor letzteres in den Fermenter eingebracht wird.

Besonders bevorzugt ist dabei vorgesehen, dass zu Zwecken der Vollauslastung dem zu vergärenden organischen Material mit geringem Anteil an organischer Trockensubstanz (oTS) weitere Biomasse aus nachwachsenden Rohstoffen, insbesondere aus Energiepflanzen, beigefügt wird.

Die mit dem erfindungsgemäßen Fermenter bzw. Verfahren produzierten Gärreste weisen einen hohen Anteil an mineralisierten Nährstoffen (N, P, K) auf und eignen sich gut als Dünger. In der Regel sind die Gärreste im Vergleich zu dem vergärenden Substrat dünnflüssiger, da sie einen geringeren Anteil an organischer Restsubstanz enthalten. Sie lassen sich daher besser ausbringen und pflanzenbaulich verwerten als z.B. Gülle. Aufgrund des reduzierten Anteils an organischer Substanz ist nach dem Austrag des Gärrestes eine sehr viel geringere Bildung von klimawirksamen Gasen, wie z.B. Kohlendioxiod ($CO_2$), Methangas ($CH_4$), und Lachgas ($N_2O$) zu befürchten. Hinzu kommt, dass ggf. in der Gülle enthaltene Pflanzensamen, insbesondere von Unkräutern, sowie Pilzsporen durch die Fermentation inaktiviert werden, und daher nach dem Austrag nicht mehr keimen können. Ein weiterer Vorteil der auf diese Weise hergestellten Gärreste ist, dass sie bei Einhaltung bestimmter Verfahrensbedingungen hygienisiert sind, und daher - ohne weitere chemische oder thermische Behandlung auch auf kritischen Flächen, wie z.B. in Wasserschutzgebieten oder auf Milchviehweiden, ausgebracht werden dürfen.

Hinzu kommt, dass die Gülle stark mineralisiert ist, die enthaltenen Nährstoffe also in wesentlich stärkerem Maße den zu düngenden Pflanzen zugute kommen. Bei unvergorener Gülle baut sich hingegen bei der Düngung ein unkalkulierbares Potenzial an organisch gebundenen Nährstoffen im Boden auf, welches bei natürlichen Mineralisierungsschüben zu erheblichen Grundwasserbelastungen führen kann, wenn die Vegetation nicht in der Lage ist, die mineralisierten Nährstoffschübe aufzunehmen..

In einer weiteren Ausgestaltung des erfindungsgemäßen Fermenters ist vorgesehen, dass dieser einem herkömmlichen Biogasfermenter nachgeschaltet ist (sogen. "Repowering"), dergestalt, dass über den Zulauf für das pumpbare organische Material Gärreste aus dem herkömmlichen Biogasfermenter einspeisbar sind.

Diese Ausgestaltung soll im folgenden als Nachgärer bezeichnet werden. Unter dem Begriff "herkömmlicher Biogasfermenter" werden die eingangs erwähnten Biogasfermenter aus dem Stand der Technik verstanden, die für die Vergärung von nachwachsenden Rohstoffen Verwendung finden und die im wesentlichen aus einem großen Rührkessel mit einer Gasspeicherkuppel oder aus einem Pfropfenströmer (einem liegenden Zylinder) bestehen. Da diese die Rohstoffe nur unvollständig vergären, gleichzeitig unter einem stetigen Verlust an methanbildenden Mikroorganismen leiden und überdies Gärreste erzeugen, die nicht ausreichend hygienisiert sind (Kurzschlußströme, siehe oben) und überdies Klimawirksame Gase emittieren (Methan, Lachgas, $CO_2$, siehe oben), ist es äußerst vorteilhaft, die Gärreste eines solchen Fermenters in den erfindungsgemäßen Fermenter einzuspeisen, der in diesem Fall als eine Art Nachgärer fungiert. Der Betreiber eines herkömmlichen Biogasfermenters kann so die Wirtschaftlichkeit und die Umweltfreundlichkeit seiner Anlage mit relativ geringen Investitionen nachhaltig verbessern.

In dieser Ausführungsform kann insbesondere auch vorgesehen sein, dass das im Nachgärer entstandene Biogas dem Gasspeicher der Hauptanlage zugeführt wird.

In einer besonders bevorzugten Ausgestaltung ist dabei vorgesehen, dass der Rückgewinnungsabschnitt dergestalt ausgebildet ist, dass die darin zurückgewonnenen spezifisch leichteren Fraktionen des pumpbaren organischen Materials dem vorgeschalteten Biogasfermenter erneut zugeführt werden können.

Es bietet sich so die Möglichkeit, die aktive Biomasse, die in Vorrichtungen nach dem Stand der Technik zusammen mit dem ausgegorenen Material in ein Gärrückstandslager verbracht wird und dort ungenutzt verbleibt, zurückzugewinnen und dem Gärprozess erneut zuzuführen. Auf diese Weise wird der Ertrag wesentlich erhöht. Hinzu kommt, dass durch die Rückführung der aktiven Biomasse in den Gärraum der Zeitraum zur Optimierung der Anlage im Erstbetrieb wesentlich verkürzt wird. Grundsätzlich ist es so, dass eine Biogasanlage eine gewisse Zeit zur Optimierung braucht. Ursache hierfür ist, dass sich in der Anlage zunächst eine stabile Mikroorganismen-Flora einstellen muss. Durch die Möglichkeit, die in dem aus dem Gärraum entnommenen, durchgegorenen Material noch befindlichen Mikroorganismen zurück zu gewinnen, wird der Zeitraum bis zum Aufbau einer stabilen und Mikroflora erheblich verkürzt. Auf diese Weise wird der maximale Ertrag also wesentlich schneller erreicht.

Die Möglichkeit der Rückführung der aktiven Biomasse weist noch einen anderen Vorteil auf: Da die Besatzdichte an aktiven Mikroorganismen im Gärraum auf einem wesentlich höheren Niveau gehalten werden kann, wird der Gärprozess beschleunigt. Daher kann der Durchsatz des Fermenters erhöht werden. So kann ein erfindungsgemäßer Fermenter wesentlich höhere Raumbelastungen tolerieren.

Ferner kann vorgesehen sein, dass der erfindungsgemäße Fermenter - alternativ oder zusätzlich zu der zuvor erwähnten Ausgestaltung - einem Langzeithydrolysereaktor (mit Flüssigkonservierung der Substrate) nachgeschaltet ist, wie er z.B. unter dem Begriff "LIGAVATOR" oder "BETAVATOR" bekannt ist. Ein solcher Reaktor hat z.B. ein Volumen von 1.500 m$^3$. Während der Lagerung des zu fermentierenden Gutes in einem solchen Reaktor findet ein anaerober Gärprozess statt (insbesondere eine Silierung, d.h. eine Milch-/Essigsäuregärung, bei der es zur Bildung von kurzkettigen Metaboliten (insbesondere Lactat, d.h. Milchsäure und Acetat, d.h. Essigsäure), zur Absenkung des pH und zur Bildung von $CO_2$ kommt. Insbesondere die besagten Milch- und Essigsäure können in dem erfindungsgemäßen Fermenter besonders gut verstoffwechselt werden. Das freiwerdende $CO_2$ kann ebenso in den erfindungsgemäßen Fermenter eingespeist werden.

**Zeichnungen und Beispiele**

Die vorliegende Erfindung wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken.

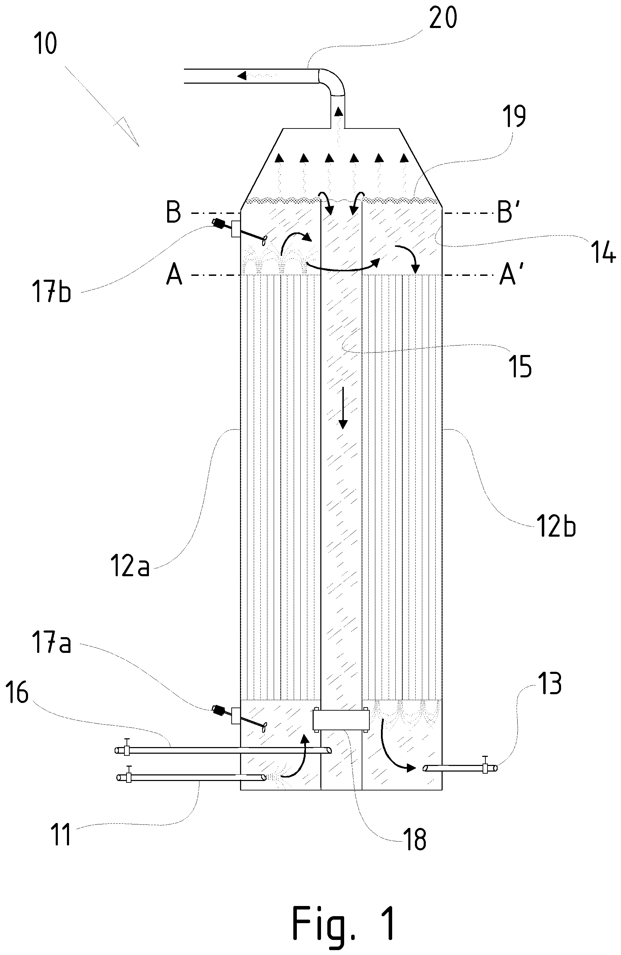

Fig. 1 zeigt eine beispielhafte Ausführungsform eines erfindungsgemäßen Fermenters 10 zur Erzeugung von Biogas aus pumpbarem organischen Material im Längsschnitt. Der Fermenter weist einen Zulauf 11 für das pumpbare organische Material, einen Festbettreaktor 12 für das pumpbare organische Material mit mindestens einem primären (aufsteigenden) Abschnitt 12a und einem sekundären (absteigenden) Abschnitt 12b, sowie mindestens einen Ablauf 13 für den entstehenden Gärrest auf.

Weiterhin weit der Fermenter eine Absetzraum 14 für das pumpbare organische Material auf, der zwischen dem primären und sekundären Abschnitt 12a, 12b des Festbettreaktors angeordnet ist, sowie einen Rückgewinnungsabschnitt 15, der mit dem Absetzraum 14 in Verbindung steht und so ausgebildet ist, dass spezifisch leichtere Fraktionen des pumpbaren organischen Materials zurückgewonnen und ggf. dem aufsteigenden (primären) Abschnitt des Festbettreaktors erneut zugeführt werden können

Der Festbettreaktor 12 weist ein Material auf, das die Ausbildung von im wesentlichen longitudinal angeordneten Kanälen (Analogie zu parallel angeordneten Darmröhren, d.h. Darmpakete) ermöglicht.

Durch die Verwendung eines Festbettreaktors mit diesen Eigenschaften werden eine Reihe von Vorteilen erzielt. So macht ein Festbettreaktor ein eigenes Rührwerk verzichtbar, wie dies in Rührkesselfermentern Verwendung findet, da sich innerhalb des Festbettreaktors ein gerichteter Materialstrom einstellen lässt. Durch den gerichteten Materialstrom werden insbesondere auch die in Rührkesselfermentern unvermeidlichen Kurzschlussströme verhindert, die eine effektive Hygienisierung des Gärmaterials sowie eine optimale Vergärung beeinträchtigen. Hinzu kommt, dass der vorgesehene Festbettreaktor ein Besiedlungssubstrat für methanbildende Mikroorganismen bereitstellt. Auf diese Weise können sich im Unterschied zu einem Rührkesselfermenter geschichtete Mikrobiozönosen einstellen.

Der Rückgewinnungsabschnitt 15 besteht aus einem senkrecht angeordneten röhrenförmigen Element und ist zwischen dem aufsteigenden (primären) Abschnitt 12a und dem absteigenden (sekundären) Abschnitt 12b des Festbettreaktors angeordnet.

Der Rückgewinnungsabschnitt 15 steht über eine Überlaufkante mit dem Absetzraum 14 in Verbindung und ist so ausgebildet, dass spezifisch leichtere Fraktionen 19 des pumpbaren organischen Materials zurückgewonnen und dem aufsteigenden (primären) Abschnitt des Festbettreaktors über einen Ablauf 16 erneut zugeführt werden können. Bei diesen spezifisch leichteren Fraktionen handelt es sich einerseits um spezifisch leichtere organische Fraktionen, wie z.B. flüchtige Fettsäuren. In herkömmlichen Rührkesselfermentern bilden diese Fraktionen eine Schwimmschicht aus und entziehen sich auf dieser Weise dem Vergärungsprozess. Sie gehen im Übrigen z.T. leicht in die Gasphase über und werden dadurch dem weiteren Vergärungsprozess dauerhaft entzogen.

Weiterhin sind in diesen spezifisch leichteren organischen Fraktionen Mikroorganismen (sog. "aktive Biomasse") enthalten, die sich vom Substrat des Festbettreaktors gelöst haben und ohne einen Rückgewinnungsabschnitt mit dem Gärrest aus dem Fermenter ausgetragen werden würden. Hierdurch wird in herkömmlichen Biogasfermentern die Besatzdichte an Biogas erzeugenden Mikroorganismen stetig reduziert, so dass diese Fermenter grundsätzlich mit einer für eine optimale Biogasausbeute zu geringen Mikroorganismendichte betrieben werden müssen. Der erfindungsgemäße Rückgewinnungsabschnitt erlaubt die Rückgewinnung und erneute Einspeisung dieser Mikroorganismen in den Fermenter, so dass der erfindungsgemäße Fermenter eine erheblich höhere Mikroorganismendichte aufweist als ein herkömmlicher Fermenter.

Weiterhin weist der Fermenter zwei verhältnismäßig klein dimensionierte Rührwerke 17a, 17b im Bereich des Zulaufs 11 und im Bereich des Absetzraums 14 auf, die in regelmäßigen Abständen eingeschaltet werden und ggf. das Absetzen fester Partikel verhindern.. Die gezeigten Rührwerke weisen im Vergleich zu den aus den herkömmlichen Rührkesselfermentern bekannten Rührwerken eine erheblich geringere Dimensionierung und Leistungsaufnahme auf.

Der Fermenter weist überdies eine Pumpe 18 zum Pumpen des Gärmaterials durch den Festbettreaktor auf. Auch diese Pumpe weist im Vergleich zu den aus den herkömmlichen Rührkesselfermentern bekannten Rührwerken eine erheblich geringere Leistungsaufnahme auf. Es kann sich insbesondere um eine Doppelkolbenpumpe handeln. In Fig. 1 ist ferner eine Gasentnahmeeinrichtung 20 zur Entnahme des erzeugten Biogases dargestellt.

Die durchgezogenen Pfeile geben die Richtung des Materialstroms durch den Fermenter an. Die unterbrochenen Pfeile geben die Richtung des entstehenden Biogases an.

Es ist in Fig. 1 auch gut zu erkennen, dass der erfindungsgemäße Fermenter einen erheblich geringeren Flächenbedarf aufweist als ein herkömmlicher Rührkesselfermenter, der wegen der großen Faulraumvolumina einen sehr hohen Flächenbedarf hat. Der erfindungsgemäße Fermenter weist in einer bevorzugten Ausgestaltung einen Flä-chenbedarf von nur 29 m$^2$ Grundfläche auf und kann so ideal in vorhandene landwirtschaftliche Hofstellen integriert werden.

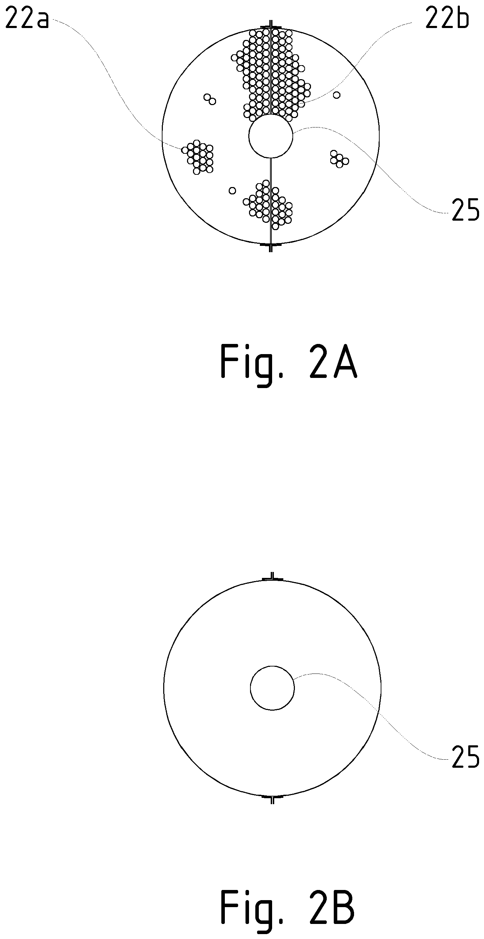

Fig. 2 zeigt zwei Querschnitte des erfindungsgemäßen Fermenters entlang der Linien A-A' (Fig. 2A) bzw. B-B' (Fig. 2B). In Fig. 2a sind in Draufsicht der aufsteigende (primäre) Abschnitt 22a und der absteigende (sekundäre) Abschnitt 22b des Festbettreaktors sowie der Rückgewinnungsabschnitt 15 zu erkennen. In Fig. 2b ist lediglich der obere Rand des Rückgewinnungsabschnitts in Aufsicht auf die Überlaufkante zu erkennen.

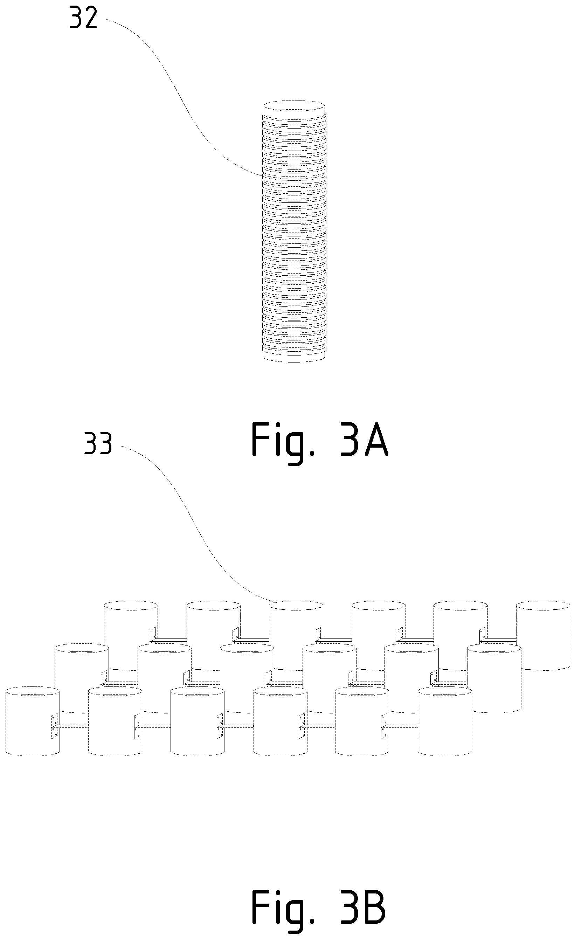

Fig. 3a zeigt beispielhaft ein Kunststoffrohr 31, das als Material für den Festkörperreaktor bevorzugt Verwendung findet, da es die Ausbildung von im wesentlichen longitudinal angeordneten Kanälen ermöglicht. Dieses Rohr weist sowohl an der Außen- wie auch an der Innenseite eine vergrößerte Oberfläche auf, die eine große Besiedlungsoberfläche für Mikroorganismen bereitstellt. Bei dem Kunststoffrohr handelt es sich um ein Rohr, das ähnliche Eigenschaften wie die bekannten flexiblen Tiefbau-Drainagerohre mit Durchmessern zwischen 50 und 400 mm aufweist. Besonders bevorzugt handelt es sich sogar um einen solchen Drainagerohrtyp, da dieser leicht und kostengünstig ist. Bevorzugt ist vorgesehen, dass viele dieser Rohre in den Fermenter eingehängt werden und so den Festbettreaktor ausbilden. Dazu kann vorgesehen sein, dass der Fermenter in seinem oberen und seinem unteren Bereich jeweils eine Aufhängungseinrichtung zum Aufhängen der besagten Kunststoffrohre aufweist.

Weitere Materialien zur Ausbildung des Festbettreaktors sind z.B. senkrecht angeordnete Röhren oder wabenartige Hohlkörper aus Keramik, Ton, Steingut, Holz, Metall oder Kunststoff, oder senkrecht angeordnete Stäbe, Seile Leinen oder Schnüre.

Fig. 3b zeigt exemplarisch eine Halteeinrichtung 33 für diese Kunststoffrohre, die am jeweils oberen und unteren Ende des Festbettreaktors angeordnet ist, und den bestmöglichen Abstand der Rohre zueinander fixiert und die Rohrdurchgänge nicht verengt, bzw. solchen Verengungen vorbeugt. Diese Halteeinrichtung besteht aus Edelstahlrohrabschnitten ("Manschetten"), die in einer Fläche angeordnet und über Winkel miteinander verschweißt oder anderweitig verbunden sind, und in welche die Enden der Kunststoffrohre passgenau gesteckt werden.

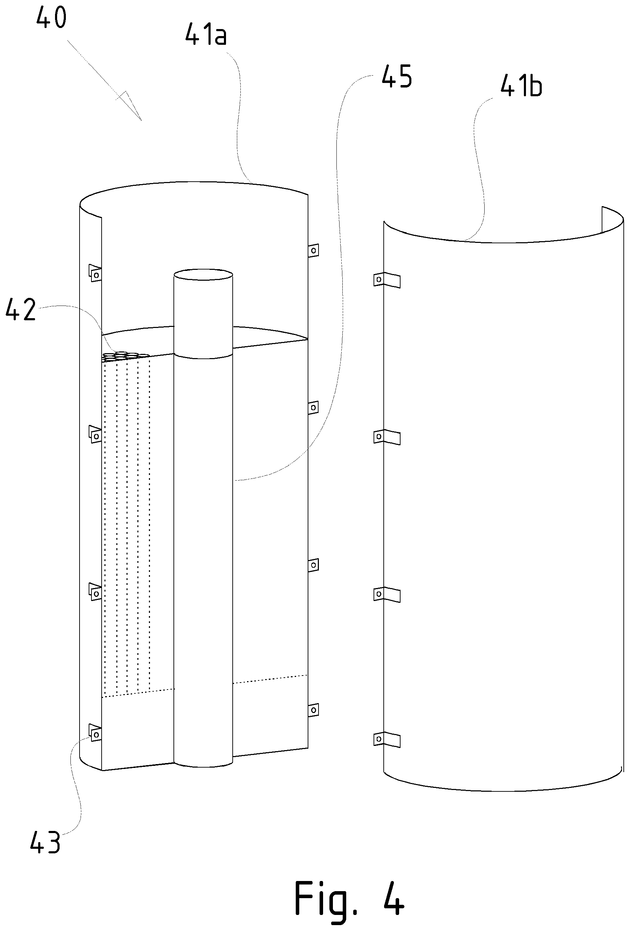

Fig. 4 zeigt einen Teil 40 eines erfindungsgemäßen Fermenters in Explosionsansicht, bestehend aus den beiden zylinderhälftenförmigen Segmenten 41a und 41b. Fig. 4 zeigt weiterhin in Draufsicht den aufsteigenden (primären) Abschnitt 42 des Festbettreaktors. Der absteigende Abschnitt ist in Fig. 4 durch die Wand des Segments 41b verdeckt und daher nicht erkennbar. Die Segmente 41a und 41b werden an Ort und Stelle über Befestigungswinkel 43 miteinander verschraubt. Idealerweise weist eines der Segmente (hier 41 a) bereits den Rückgewinnungsabschnitt 45 auf, was darüber hinaus die Fertigung und Montage erleichtert und so Kosten reduziert.

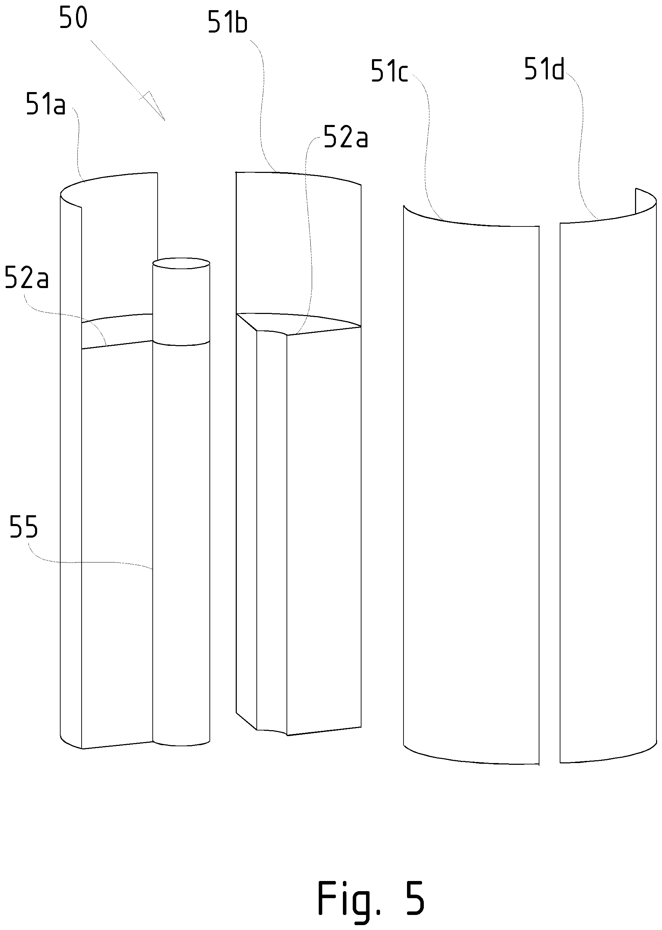

Fig. 5 zeigt ebenfalls einen Teil 50 eines erfindungsgemäßen Fermenters in Explosionsansicht. Dieser besteht im Unterschied zu dem in Fig. 4 gezeigten Fermenter aus vier Segmenten 51a - 51d. Der aufsteigende (primäre) Abschnitt des Festbettreaktors besteht folglich aus zwei Segmenten 52a und 52 b. Der absteigende Abschnitt ist in Fig. 5 durch die Wand der Segmente 51c und 51d verdeckt und daher nicht erkennbar. Ferner ist der Rückgewinnungsabschnitt 55 dargestellt.

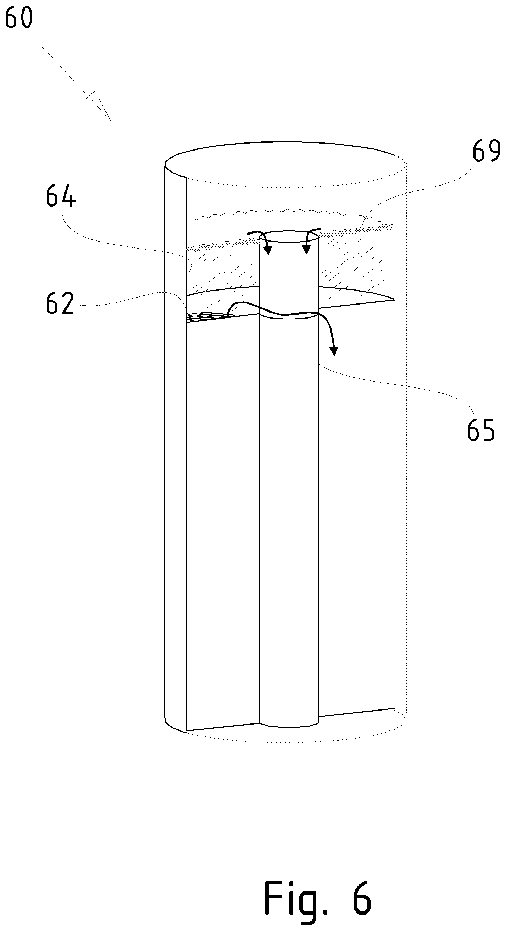

Fig. 6 zeigt ebenfalls einen Teil 60 eines erfindungsgemäßen Fermenters im Anschnitt mit den Strömungsverläufen des Gärmaterials. Das Gärmaterial tritt aus dem aufsteigenden (primären) Abschnitt 62 des Festbettreaktors in den Absetzraum 64. Dort setzen sich spezifisch leichtere Fraktionen 69 nach oben ab und gelangen über eine Überlaufkante in den Rückgewinnungsabschnitt 65. Die spezifisch schwereren Fraktionen (z.B. nicht mehr Gas bildende abgestorbene Biomasse) gelangen hingegen in den nicht dargestellten absteigenden (sekundären) Abschnitt des Festbettreaktors.

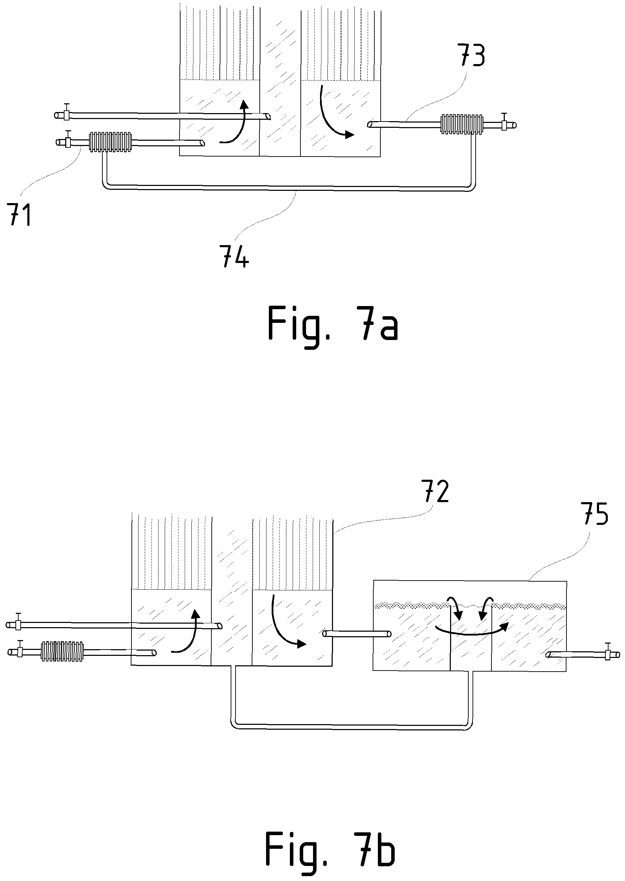

Fig. 7 zeigt verschiedene ergänzende Ausgestaltungen des erfindungsgemäßen Fermenters. So ist in Fig. 7a ein im Bereich seines Ablaufs 73 angeordneter Wärmetauscher 74 dargestellt, mit welchem das frische zu fermentierende organische Material vorwärmbar ist. Zu diesem Zweck steht der Wärmetauscher mit dem Zulauf 71 in Verbindung.

Auf diese Weise wird die Einstellung meso- oder thermophiler Bedingungen im Fermenter erheblich erleichtert und gleichzeitig der hierfür erforderliche Energieaufwand reduziert. Im Idealfall reicht die intrinsische, sich bei der Vergärung entwickelnde Reaktionswärme zur Einstellung der genannten Bedingungen aus, so dass eine Wärmezufuhr von Außen überflüssig ist.

Fig. 7b zeigt einen weiteren Rückgewinnungsabschnitt 75, der nach dem absteigenden (sekundären) Abschnitt des Festbettreaktors 72 angeordnet ist. Auf diese Weise wird die Rückgewinnung der genannten Substrate und Mikroorganismen nochmals verbessert.

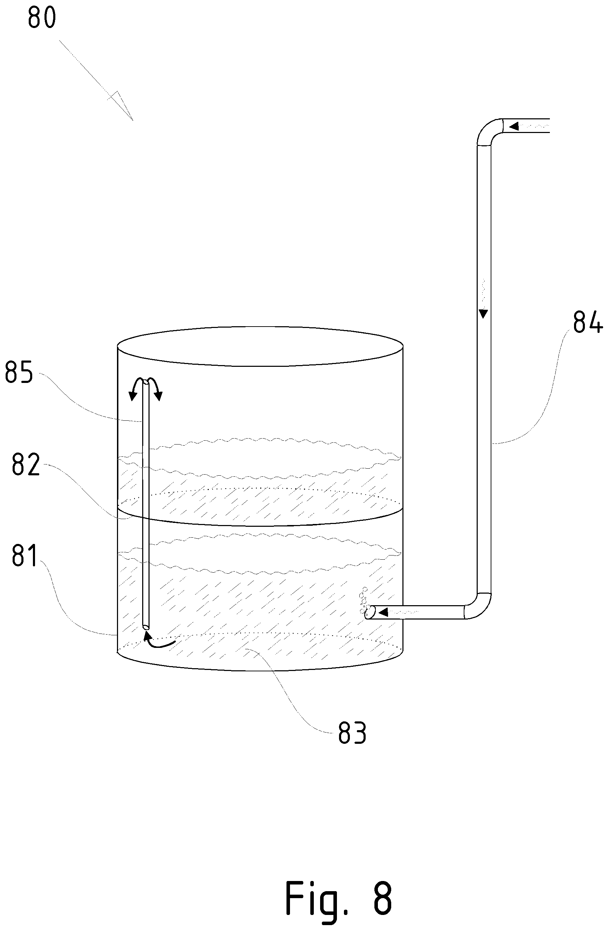

Fig. 8 zeigt eine hydrostatische Gasspeichereinrichtung 80, bestehend aus einem Behälter 81 mit einem Zwischenboden 82. Der untere Teil des Behälters ist mit einer Sperrflüssigkeit 83 gefüllt. Die Gasspeichereinrichtung ist an eine Gasentnahmeeinrichtung 84 eines nicht dargestellten Fermenters angeschlossen. Das eingeleitete Gas verdrängt beim Einströmen in den unteren Teil des Behälters die Flüssigkeit (insbesondere Wasser) gegen die Schwerkraft (und damit gegen einen sich aufbauenden hydrostatischen Druck bzw. eine Wassersäule). Das Wasser weicht durch ein Steigrohr 85 in den oberen Teil des Behälters aus. Ist die Gasspeichereinrichtung z.B. so konstruiert, dass das einströmende Gas bei dem Verdrängen der darin befindlichen Flüssigkeit eine maximale Wassersäule von 2000 mm aufbaut, so entspricht dies einem hydrostatischen Druck von 200 mbar. Gleichzeitig wird das gespeicherte Gas auf einem diesem Druck entsprechenden Gasdruck gehalten, und kann unter Verzicht auf ein eigenes Gasdruckgebläse dem Gasmotor des BHKW zugeführt werden. Entscheidend hierfür ist, dass die Biogas erzeugenden Mikroorganismen auch gegen starke Druckgradienten weiterhin Biogas erzeugen können. In der Literatur sind hier Druckgradienten bis zu 160 bar beschrieben. Der beschriebene, sich aufbauende Druckgradient von 200 mbar, der sich u.U. bis in den Fermenter fortsetzt, beeinträchtigt die Biogassynthese also nicht.

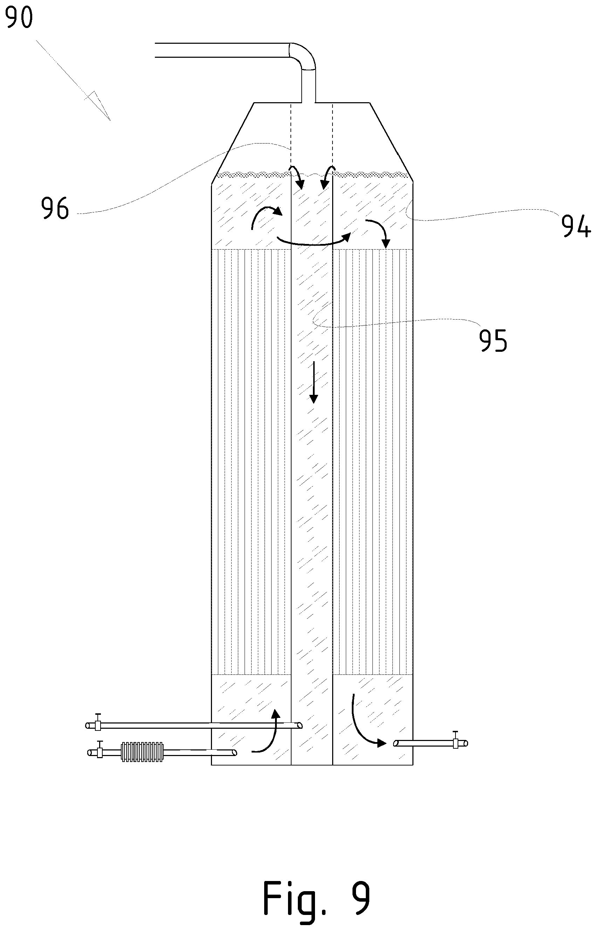

Fig. 9 zeigt eine weitere Ausführungsform eines erfindungsgemäßen Fermenters 90, die in den meisten Punkten der in Fig. 1 gezeigten Ausführungsform entspricht. Im Unterschied zu lezterer ist hier jedoch vorgesehen, dass der Rückgewinnungsabschnitt 95 über periphere Bohrungen 96 bzw. Siebeinrichtungen mit dem Absetzraum 94 in Verbindung steht, und nicht über eine Überlaufkante.

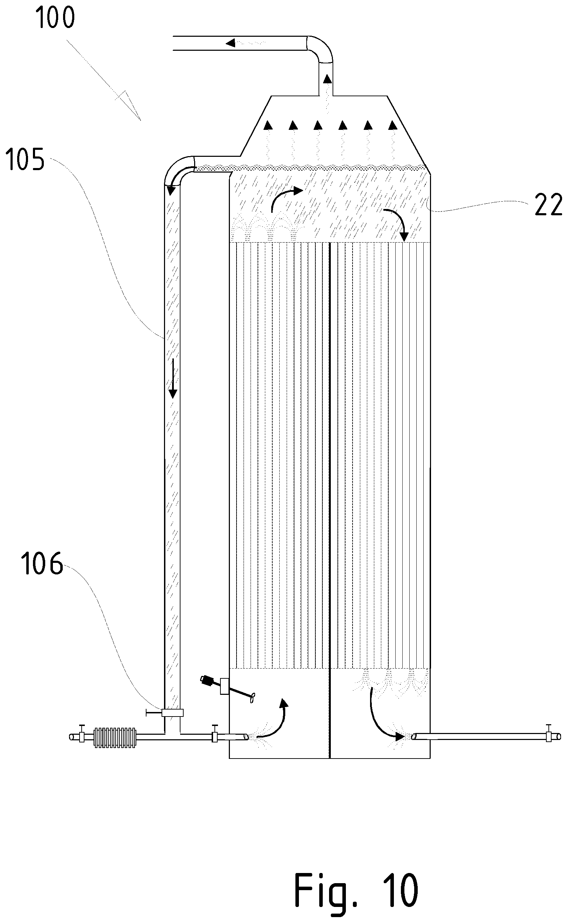

Fig. 10 zeigt eine weitere Ausführungsform eines erfindungsgemäßen Fermenters 100, die ebenfalls in den meisten Punkten der in Fig. 1 gezeigten Ausführungsform entspricht. Im Unterschied zu Letzterer ist hier jedoch vorgesehen, dass der Rückgewinnungsabschnitt 105 extern angeordnet ist und mit dem Absetzraum 104 in Verbindung steht. Über ein Ventil 106 kann die Menge des Rücklaufs reguliert werden. Der Rückgewinnungsabschnitt kann in dieser Bauweise mitsamt allen leicht zu Wartungszwecken vom Fermenter getrennt werden.

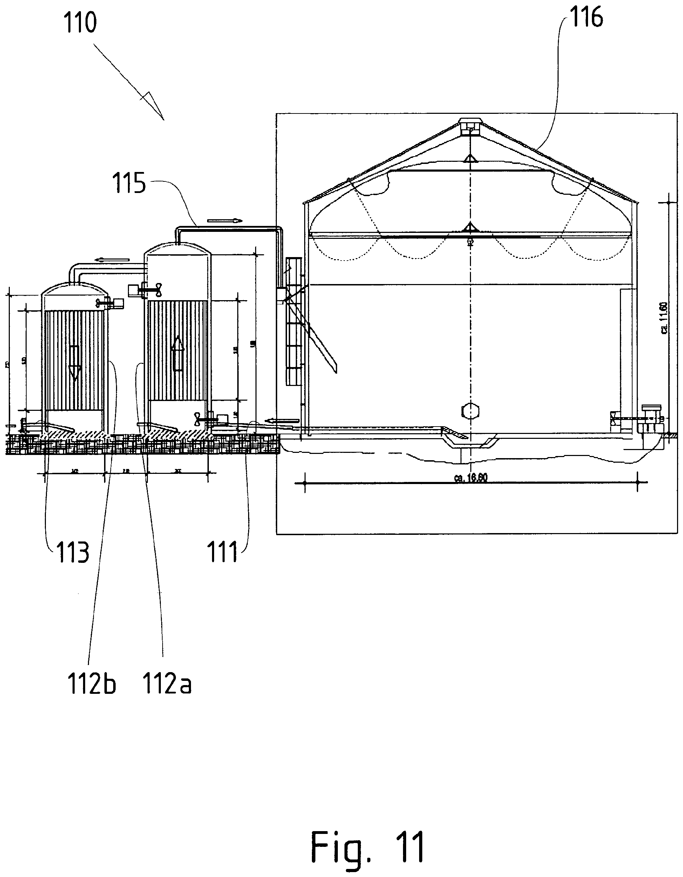

Fig. 11 zeigt eine weitere Ausführungsform des erfindungsgemäßen Fermenters mit dem Zulauf 111, einen Festbettreaktor 112 für das pumpbare organische Material mit einem primären (aufsteigenden) Abschnitt 112a und einem sekundären (absteigenden) Abschnitt 112b, die räumlich und baulich voneinander getrennt sind (sogenannte Teilfermenter), sowie einem Ablauf 113. Die Abschnitte 112a und 112b können aus einem kostengünstig verfügbaren, gebrauchten Flüssiggas- oder Gastank gefertigt sein, der etwa mittig durchgetrennt wurde. Ein Rückgewinnungsabschnitt 115, über welchen die zurückgewonnenen spezifisch leichteren Fraktionen des pumpbaren organischen Materials dem vorgeschalteten Biogasfermenter 116 erneut zugeführt werden können, ist im Kopfbereich der Teilfermenter ebenfalls vorgesehen. Die Förderung des Materials erfolgt dabei insbesondere über den im aufsteigenden Abschnitt des Fermenters entstehenden Gasdruck.

Fig. 11 zeigt weiterhin einen optional vorgesehenen herkömmlichen Biogasfermenter 116, dem der erfindungsgemäße Fermenter 110 nachgeschaltet ist und diesem gegenüber als Nachgärer fungiert (sogen. "Repowering"), dergestalt, dass über den Zulauf 111 Gärreste aus dem herkömmlichen Biogasfermenter einspeisbar sind.

Der Biogasfermenter 116 besteht im Wesentlichen aus einem großen Rührkessel mit einer Gasspeicherkuppel. Da dieser die Rohstoffe nur unvollständig vergärt (d.h. hohes Restgaspotenzial des Gärrests), gleichzeitig unter einem stetigen Verlust an methanbildenden Mikroorganismen leidet und überdies Gärreste erzeugt, die nicht ausreichend hygienisiert und mineralisiert (ausgefault) sind (Kurzschlußströme, siehe oben) und überdies Klimawirksame Gase emittiert (Methan , Lachgas, $CO_2$, siehe oben), ist es äußert vorteilhaft, die Gärreste eines solchen Fermenters 116 in den erfindungsgemäßen Fermenter 112 einzuspeisen. Der Betreiber eines herkömmlichen Biogasfermenters kann so die Wirtschaftlichkeit und die Umweltfreundlichkeit seiner Anlage mit relativ geringen Investitionen nachhaltig verbessern.

Das im Fermenter 112 entstandene Biogas wird dabei dem Gasspeicher des Biogasfermenters 116 zugeführt wird. In einer besonders bevorzugten Ausgestaltung ist dabei vorgesehen, dass der Rückgewinnungsabschnitt 115 dergestalt ausgebildet ist, dass die darin zurückgewonnenen spezifisch leichteren Fraktionen des pumpbaren organischen Materials dem vorgeschalteten Biogasfermenter 116 erneut zugeführt werden können.

**Patentansprüche**

1. Verfahren zur Erzeugung von Biogas aus pumpbarem organischen Material mit geringem Anteil an organischer Trockensubstanz in einem Fermenter,
   wobei das Verfahren die folgenden Schritte aufweist:

   i) Einbringen des pumpbaren organischen Materials durch einen Zulauf in den Fermenter,
   ii) Erzeugung und Aufrechterhaltung eines anaeroben Milieus, eines pH-Werts von mindestens 7 und einer Temperatur im mesophilen bis thermophilen Bereich,
   iii) Erzeugung eines Materialstroms des pumpbaren organischen Materials durch einen Festbettreaktor sowie einen Absetzraum des Fermenters,
   iv) Rückgewinnung der spezifisch leichteren Fraktionen des pumpbaren organischen Materials in einem Rückgewinnungsabschnitt,
   v) sowie Auffangen des entstehenden Gases und kontinuierliche oder schubweise Entnahme des vergorenen Gärrestes.

2. Fermenter geeignet zur Durchführung des Verfahrens zur Erzeugung von Biogas aus pumpbarem organischen Material mit geringem Anteil an organischer Trockensubstanz gemäß Anspruch 1, aufweisend

   a) mindestens einen Zulauf für das pumpbare organische Material,
   b) mindestens einen Festbettreaktor für das pumpbare organische Material mit mindestens einem primären und einem sekundären Abschnitt, sowie

c) mindestens einen Ablauf für den entstehenden Gärrest,
wobei dieser außerdem

   d) mindestens einen Absetzraum für das pumpbare organische Material aufweist, der zwischen dem primären und sekundären Abschnitt des Festbettreaktors angeordnet ist, sowie
   e) mindestens einen Rückgewinnungsabschnitt, der mit dem Absetzraum in Verbindung steht und so ausgebildet ist, dass spezifisch leichtere Fraktionen des pumpbaren organischen Materials zurückgewonnen werden können.

3.  Fermenter gemäß Anspruch 2, **dadurch gekennzeichnet, dass** der primäre Abschnitt des Festbettreaktors ein aufsteigender Abschnitt und der sekundäre Abschnitt des Festbettreaktors ein absteigender Abschnitt ist.

4.  Fermenter gemäß einem der Ansprüche 2 - 3, **dadurch gekennzeichnet, dass** der Festbettreaktor ein Material aufweist, das eine große Besiedlungsoberfläche für Mikroorganismen zur Verfügung stellt.

5.  Fermenter gemäß einem der Ansprüche 2 - 4, **dadurch gekennzeichnet, dass** der Festbettreaktor ein Material aufweist, das die Ausbildung von im Wesentlichen longitudinal angeordneten Kanälen ermöglicht.

6.  Fermenter gemäß einem der Ansprüche 2 - 5, **dadurch gekennzeichnet, dass** der Rückgewinnungsabschnitt zwischen dem aufsteigenden (primären) und dem absteigenden (sekundären) Abschnitt mindestens eines Festbettreaktors angeordnet ist.

7.  Fermenter gemäß einem der Ansprüche 2 - 6, **dadurch gekennzeichnet, dass** ein weiterer Rückgewinnungsabschnitt nach dem absteigenden (sekundären) Abschnitt des Festbettreaktors angeordnet ist.

8.  Fermenter gemäß einem der Ansprüche 2 - 7, **dadurch gekennzeichnet, dass** dieser die äußere Form eines stehend angeordneten Zylinders aufweist

9.  Fermenter gemäß einem der Ansprüche 2 - 8, **dadurch gekennzeichnet, dass** dieser aus mehreren Segmenten besteht, die in einem Fertigungsbetrieb herstellbar sind und an Ort und Stelle zu einem Fermenter zusammenfügbar sind.

10. Fermenter gemäß einem der Ansprüche 2 - 9, **dadurch gekennzeichnet, dass** dieser eine zumindest teilweise oberhalb des Festbettreaktors und/oder der Rückgewinnungseinrichtung angeordnete Gasauffangeinrichtung aufweist.

11. Fermenter gemäß einem der Ansprüche 2 - 10, **dadurch gekennzeichnet, dass** dieser eine hydrostatische Gasspeichereinrichtung aufweist.

12. Fermenter gemäß einem der Ansprüche 2 - 11, **dadurch gekennzeichnet, dass** die Gasauffangeinrichtung eine kegelförmige, kegelstumpfförmige, paraboloide oder hemispärische Kuppel aufweist.

13. Fermenter gemäß einem der Ansprüche 2 - 12, **dadurch gekennzeichnet, dass** dieser im Bereich seines Ablaufs einen Wärmetauscher aufweist, mit welchem das frische zu fermentierende organische Material vorwärmbar ist.

14. Fermenter gemäß einem der Ansprüche 2 - 13, **dadurch gekennzeichnet, dass** der Fermenter eine Temperierungseinrichtung für das zu vergärende organische Material aufweist, die so eingerichtet ist, dass die Temperatur das Gärmaterials im Gärraum allein durch die Temperierung des über den Zulauf eingebrachten, zu vergärenden organischen Materials einstellbar ist.

15. Verfahren gemäß Anspruch 1, **dadurch gekennzeichnet, dass** das rückgewonnene Material mit frischem, zu vergärenden Material vorinkubiert wird, bevor letzteres in den Fermenter eingebracht wird.

16. Verfahren gemäß Anspruch 1 oder 15, **dadurch gekennzeichnet, dass** zu Zwecken der Vollauslastung dem zu vergärenden organischen Material weitere Biomasse, z. B. aus nachwachsenden Rohstoffen, insbesondere aus Energiepflanzen, beigefügt wird.

17. Verfahren gemäß Anspruch 1 oder 15 - 16, **dadurch gekennzeichnet, dass** die Verfahrensbedingungen so eingestellt sind, dass die Bildung von Propionsäure vermindert bzw. Propionsäure verstärkt abgebaut wird.

18. Fermenter gemäß einem der Ansprüche 2 - 14, **dadurch gekennzeichnet, dass** dieser einem herkömmlichen Biogasfermenter nach geschaltet ist, dergestalt, dass über den Zulauf für das pumpbare organische Material Gärreste aus dem herkömmlichen Biogasfermenter einspeisbar sind.

19. Fermenter gemäß Anspruch 18, **dadurch gekennzeichnet, dass** der Rückgewinnungsabschnitt dergestalt ausgebildet ist, dass die darin zurückgewonnenen spezifisch leichteren Fraktionen des pumpbaren organischen Materials dem vorgeschalteten Biogasfermenter erneut zugeführt werden können.

20. Fermenter gemäß einem der Ansprüche 2 - 14 oder 18 - 19, **dadurch gekennzeichnet, dass** dieser einem Langzeithydrolysereaktor nachgeschaltet ist.

21. Verfahren nach einem der Ansprüche 1 oder 15 - 17, **dadurch gekennzeichnet, dass** nach Schritt iv) des Verfahrens der Schritt der erneuten Zuführung des rückgewonnenen Materials zu dem Fermenter erfolgt

22. Fermenter gemäß einem der Ansprüche 2 - 14 oder 18 - 19, **dadurch gekennzeichnet. dass** der Rückgewinnungsabschnitt über eine Überlaufkante mit dem Absetzraum in Verbindung steht.

## Claims

1. Method for generating biogas from pumpable organic material with a low content of organic dry substance in a fermenter, said method comprising following steps:

   i) placing the pumpable organic material through an inlet into the fermenter,
   ii) generating and maintaining an anaerob environment, a pH of at least 7 and a temperature in the mesophil to thermophil range,
   iii) generating a transfer stream of the pumpable organic material through a fixed bed reactor and a sedimentation chamber of the fermenter,
   iv) recovering the specific lighter fractions of the pumpable organic material in a recycling section,
   v) and collecting the rising gases and continuously or periodically removing the remaining fermented leftovers.

2. Fermenter suitable for performing the method for generating biogas from pumpable organic material with a low content of organic dry substance according to claim 1, comprising

   a) at least one inlet for the pumpable organic material,
   b) at least one fixed bed reactor for the pumpable organic material comprising at least one primary and one secondary section, and
   c) at least one outlet for the remaining fermentation leftovers,
   further comprising

   d) at least one sedimentation chamber for the pumpable organic material, arranged between the primary and secondary sections of the fixed bed reactor, and
   e) at least one recycling section connected to the sedimentation chamber and designed such that specific lighter fractions of the pumpable organic material can be recovered.

3. Fermenter according to claim 2, **characterized in that** the primary section of the fixed bed reactor is an ascending section and the secondary section of the fixed bed reactor is a descending section.

4. Fermenter according to any of claims 2-3, **characterized in that** the fixed bed reactor comprises a material providing a large cultivation surface for seeding of microorganisms.

5. Fermenter according to any of claims 2-4, **characterized in that** the fixed bed reactor comprises a material allowing for the formation of essentially longitudinally arranged channels.

6. Fermenter according to any of claims 2-5, **characterized in that** the recycling section is arranged between the ascending (primary) and the descending (secondary) section of at least one fixed bed reactor.

7. Fermenter according to any of claims 2-6, **characterized in that** a further recycling section is arranged subsequently to the descending (secondary) section of the fixed bed reactor.

8. Fermenter according to any of claims 2-7, **characterized in that** the same comprises the outer shape of a standing cylinder.

9. Fermenter according to any of claims 2-8, **characterized in that** the same consists of several segments which can be produced in a manufacturing facility and assembled to a fermenter on site.

10. Fermenter according to any of claims 2-9, **characterized in that** the same comprises a gas collecting section arranged at least partly above the fixed bed reactor and/or the recycling section.

11. Fermenter according to any of claims 2-10, **characterized in that** the same comprises a hydrostatic gas storage section.

12. Fermenter according to any of claims 2-11, **characterized in that** the gas collecting section comprises a cone-shaped, truncated-cone-shaped, paraboloid or hemispherical dome.

13. Fermenter according to any of claims 2-12, **characterized in that** the same comprises a heat exchanger in the vicinity of its outlet serving for preheating of the fresh organic material to be fermented.

14. Fermenter according to any of claims 2-13, **characterized in that** the same comprises a temperature control system for the organic material to be fermented, designed such that the temperature of the material to be fermented in the fermentation chamber is adjusted solely by tempering the organic material to be fermented which is placed into the fermenter via the inlet.

15. Method according to claim 1, **characterized in that** the recycled material is preincubated with fresh material to be fermented before the latter is placed into the fermenter.

16. Method according to claim 1 or 15, **characterized in that** further biomass, e.g., from regenerative resources, especially from energy plants, is added to the organic material to be fermented for purpose of full utilization of the capacity.

17. Method according to claim 1 or claims 15-16, **characterized in that** process conditions are adjusted such that the formation of propionic acid is diminished or degradation of propionic acid is increased.

18. Fermenter according to any of claims 2-14, **characterized in that** the same is arranged subsequently to a conventional biogas fermenter such that remaining fermentation leftovers from the conventional biogas fermenter can be placed into said fermenter via the inlet for the pumpable organic material.

19. Fermenter according to claim 18, **characterized in that** the recycling section is arranged such that the specific lighter fractions of the pumpable organic material recycled therein can be placed back into the preceding biogas fermenter.

20. Fermenter according to any of claims 2-14 or 18-19, **characterized in that** the same is arranged subsequently to a long-term hydrolysis reactor.

21. Method according to any of claims 1 or 15-17, **characterized in that** after step iv) of the method, there is a step of adding back the recycled material to the fermenter.

22. Fermenter according to any of claims 2-14 or 18-19, **characterized in that** the recycling section is connected with the sedimentation chamber via an overflow edge.

**Revendications**

1. Procédé de production de biogaz dans un fermenteur à partir de matière organique apte au pompage ayant une faible teneur en substances organiques sèches,

le procédé présentant les étapes suivantes :

  i) introduction de la matière organique apte au pompage dans le fermenteur par une alimentation,
  ii) génération et maintien d'un milieu en anaérobie, d'une valeur de pH d'au moins 7 et d'une température située dans la plage mésophile à thermophile,
  iii) génération d'un flux de matière organique apte au pompage au moyen d'un réacteur à lit fixe ainsi que d'une chambre de décantation du fermenteur,
  iv) recyclage des fractions spécifiquement plus légères de la matière organique apte au pompage dans une section de recyclage,
  v) ainsi que captation du gaz produit et prélèvement continu ou par lots des résidus fermentés issu de la fermentation.

2. Fermenteur approprié pour la réalisation du procédé de production de biogaz à partir de matière organique apte au pompage ayant une faible teneur en substances organiques sèches selon la revendication 1, présentant

  a) au moins une alimentation pour la matière organique apte au pompage,
  b) au moins un réacteur à lit fixe pour la matière organique apte au pompage comprenant au moins une section primaire et une section secondaire, ainsi que
  c) au moins une évacuation pour les résidus fermentés produits,
  dans lequel celui-ci

  d) présente au moins une chambre de décantation pour la matière organique apte au pompage disposée entre la section primaire et la section secondaire du réacteur à lit fixe, ainsi que
  e) au moins une section de recyclage qui communique avec la chambre de décantation et qui est réalisée de telle sorte que des fractions spécifiquement plus légères de la matière organique apte au pompage peuvent être recyclées.

3. Fermenteur selon la revendication 2, **caractérisé en ce que** la section primaire du réacteur à lit fixe est une section montante et la section secondaire est une section descendante.

4. Fermenteur selon l'une des revendications 2 à 3, **caractérisé en ce que** le réacteur à lit fixe présente un matériau qui met à disposition une large surface de colonisation pour les micro-organismes.

5. Fermenteur selon l'une des revendications 2 à 4, **caractérisé en ce que** le réacteur à lit fixe présente un matériau qui permet la formation de canaux disposés essentiellement longitudinalement.

6. Fermenteur selon l'une des revendications 2 à 5, **caractérisé en ce que** la section de recyclage est disposée entre la section montante (primaire) et la section descendante (secondaire) d'au moins un réacteur à lit fixe.

7. Fermenteur selon l'une des revendications 2 à 6, **caractérisé en ce qu'**une section de recyclage supplémentaire est disposée en aval de la section descendante (secondaire) du réacteur à lit fixe.

8. Fermenteur selon l'une des revendications 2 à 7, **caractérisé en ce que** celui-ci présente la forme extérieure d'un cylindre placé en position debout.

9. Fermenteur selon l'une des revendications 2 à 8, **caractérisé en ce que** celui-ci est constitué de plusieurs segments qui peuvent être fabriqués dans un établissement industriel et assemblés sur site en un fermenteur.

10. Fermenteur selon l'une des revendications 2 à 9, **caractérisé en ce que** celui-ci présente un dispositif de captation de gaz disposé au moins en partie au-dessus du réacteur à lit fixe et/ou du dispositif de recyclage.

11. Fermenteur selon l'une des revendications 2 à 10, **caractérisé en ce que** celui-ci présente un dispositif de stockage de gaz hydrostatique.

12. Fermenteur selon l'une des revendications 2 à 11, **caractérisé en ce que** le dispositif de captation de gaz présente une coupole conique, tronconique, paraboloïde ou hémisphérique.

13. Fermenteur selon l'une des revendications 2 à 12, **caractérisé en ce que** celui-ci présente un échangeur de chaleur, dans le voisinage de son évacuation, au moyen duquel la matière organique fraîche à fermenter peut être préchauffée.

**14.** Fermenteur selon l'une des revendications 2 à 13, **caractérisé en ce que** le fermenteur présente un dispositif d'équilibrage de la température pour la matière organique à fermenter qui est configuré de telle sorte que la température de la matière à fermenter dans la chambre de fermentation peut être réglée simplement par l'équilibrage de la température de la matière organique à fermenter amenée par l'alimentation.

**15.** Procédé selon la revendication 1, **caractérisé en ce que** la matière recyclée est pré-incubée avec de la matière fraîche à fermenter avant que cette dernière ne soit introduite dans le fermenteur.

**16.** Procédé selon la revendication 1 ou 15, **caractérisé en ce que** de la biomasse additionnelle, p. ex. issue de matières premières renouvelables, en particulier à partir de plantes énergétiques, est ajoutée à la matière organique à fermenter, à des fins d'utilisation à pleine capacité.

**17.** Procédé selon la revendication 1 ou 15 à 16, **caractérisé en ce que** les conditions de procédé sont réglées de telle sorte que la formation d'acide propionique est réduite, respectivement, que l'acide propionique est rapidement décomposé.

**18.** Fermenteur selon l'une des revendications 2 à 14, **caractérisé en ce que** celui-ci est installé en aval d'un fermenteur de biogaz conventionnel si bien qu'il est possible d'alimenter des résidus fermentés provenant du fermenteur de biogaz conventionnel au moyen de l'alimentation en matière organique apte au pompage.

**19.** Fermenteur selon la revendication 18, **caractérisé en ce que** la section de recyclage est constituée de telle sorte que les fractions spécifiquement plus légères de la matière organique apte au pompage recyclées dans la section de recyclage peuvent être alimentées de nouveau dans le fermenteur de biogaz installé en amont.

**20.** Fermenteur selon l'une des revendications 2 à 14 ou 18 à 19, **caractérisé en ce que** celui-ci est installé en aval d'un réacteur d'hydrolyse à long terme.

**21.** Procédé selon l'une des revendications 1 ou 15 à 17, **caractérisé en ce que** l'étape de réalimentation de la matière recyclée dans le fermenteur s'effectue après l'étape iv) du procédé.

**22.** Fermenteur selon l'une des revendications 2 à 14 ou 18 à 19, **caractérisé en ce que** la section de recyclage communique avec la chambre de décantation par l'intermédiaire d'un rebord de trop-plein.

Fig. 1

Fig. 2A

Fig. 2B

32

Fig. 3A

33

Fig. 3B

Fig. 4

Fig. 5

Fig. 6

73

71

74

Fig. 7a

72

75

Fig. 7b

80

85

82

81

83

84

Fig. 8

Fig. 9

100

105

22

106

Fig. 10

Fig. 11

**IN DER BESCHREIBUNG AUFGEFÜHRTE DOKUMENTE**

**In der Beschreibung aufgeführte Patentdokumente**

- DE 19756485
- DE 3604415
- EP 0335825 A1
- DE 4415017